(19) **Europäisches Patentamt / European Patent Office / Office européen des brevets**

(11) **EP 4 692 791 A1**

(12) # EUROPEAN PATENT APPLICATION
published in accordance with Art. 153(4) EPC

(43) Date of publication:
**11.02.2026 Bulletin 2026/07**

(21) Application number: **24780431.3**

(22) Date of filing: **27.03.2024**

(51) International Patent Classification (IPC):
**G01N 33/48** (2006.01)

(52) Cooperative Patent Classification (CPC):
**G01N 33/48**

(86) International application number:
**PCT/JP2024/012221**

(87) International publication number:
**WO 2024/204325 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **28.03.2023 JP 2023050776**

(71) Applicant: **TORAY INDUSTRIES, INC.
Tokyo 103-8666 (JP)**

(72) Inventors:
- **IDO Takayoshi
Kamakura-shi, Kanagawa 248-8555 (JP)**
- **OKANO Fumiyoshi
Kamakura-shi, Kanagawa 248-8555 (JP)**

(74) Representative: **Mewburn Ellis LLP
Aurora Building
Counterslip
Bristol BS1 6BX (GB)**

(54) **METHOD FOR DETECTING CELL MEMBRANE PROTEIN**

(57) The present invention addresses the problem of providing a method for staining a cell membrane protein with high accuracy. Provided is a method for detecting a cell membrane protein in an embedded fixed tissue, said method comprising an embedded medium removal step for bringing a fixed tissue including a solid embedded medium into contact with a removal solution without baking the fixed tissue, thereby removing the embedded medium.

Fig. 15

EP 4 692 791 A1

**Description**

Technical Field

The present invention relates to a method for detecting a cell membrane protein in an embedded fixed tissue, and a method for determining, using the above method, a treatment for an individual from whom a fixed tissue is derived.

Background Art

A specific cell membrane protein on a cancer cell can be used as a disease marker for the detection of a cancer since the protein is specific to the cancer. Moreover, various antibody drugs using such a protein as a target antigen have been applied as cancer therapeutic agents with fewer side effects in cancer treatment. For example, in many cancer cells, cytopasmic-activation and proliferation-associated protein 1 (CAPRIN-1) protein is expressed on the surface of the cell membrane, and the protein is known to be a promising disease marker for cancers (Patent Literature 1). Further, an antibody against CAPRIN-1 protein is known to be promising for use as a therapeutic and/or prophylactic agent for cancers (Patent Literature 2).

In recent years, studies have been carried out in an attempt to enhance the pharmacological effects of these antibody drugs, and such studies have especially focused on the development of an antibody-drug conjugate (ADC) in which a drug having a direct strong and cytocidal ability is conjugated with an antibody (Non-Patent Literature 1 and 2). Thus, antibody drugs have been actively developed, and various types of pharmaceuticals have become commercially available.

However, in the treatment using molecular-targeted drugs including these antibody drugs, the pharmacological effect largely varies among individuals, and this has been thought to be one of the major technical problems. This is largely due to the fact that the abundance ratios and the types of cell membrane proteins that serve as the antigens for antibody drugs in diseased cells such as cancer cells are largely different among individuals. Therefore, companion diagnostics, in which information obtained from a specimen obtained from a patient is used to select a treatment that is particularly effective for the patient, has recently been regarded as important. In particular, since cell membrane proteins are often used as the antigens for antibody drugs, accurate determination of the presence of a cell membrane protein in a specimen is important.

In the analysis of the protein expression in a specimen, immunohistochemical staining is often employed. In common immunohistochemical staining, an embedding medium (such as paraffin) is first melted by a method called baking, in which a specimen is heated in the atmosphere. Thereafter, the embedding medium is removed by dissolving it in an organic solvent or the like (Patent Literature 3 and 4). Further, the specimen from which the embedding medium has been removed is incubated together with an antibody to perform staining. However, a highly accurate staining method for a cell membrane protein has not yet been established.

Citation List

Patent Literature

Patent Literature 1: WO 2010/016527
Patent Literature 2: WO 2010/016526
Patent Literature 3: WO 2011/025442
Patent Literature 4: WO 2004/077057

Non-Patent Literature

Non-Patent Literature 1: Lancet Oncol 2016; 17: e256-62
Non-Patent Literature 2: Pharm Res. 2015 Nov; 32(11): 3526-40

Summary of Invention

Technical Problem

An object of the present invention is to provide a highly accurate staining method for a cell membrane protein.

Solution to Problem

The present inventors intensively studied to find that the accuracy of staining of a cell membrane protein can be remarkably improved by eliminating the operation of baking, which has not been considered to have an adverse effect on the result, thereby completing the present invention.

Specifically, the present invention has the following characteristics [1] to [21].

[1-1] A method for detecting a cell membrane protein in an embedded fixed tissue, comprising: an embedding medium removal process of removing an embedding medium by contacting the fixed tissue comprising the embedding medium in a solid state with a removal solution; an antigen retrieval process of retrieving the cell membrane protein in the fixed tissue after the embedding medium removal process; a primary antibody reaction step of reacting a primary antibody having immunological reactivity against the cell membrane protein with the cell membrane protein of the fixed tissue; a secondary antibody reaction step of reacting a labeled secondary antibody having immunological reactivity against the primary antibody with the fixed tissue after the primary antibody reaction step; and a label detecting process of detecting the labeled secondary antibody bound to the cell membrane protein through the primary antibody after the secondary antibody reaction step.

[1-2] A method for detecting a cell membrane protein by removing an embedding medium from an embedded fixed tissue, comprising: an embedding medium removal process of removing the embedding medium by contacting the fixed tissue comprising the embedding medium in a solid state with a removal solution; a labeling process of binding the cell membrane protein of the fixed tissue after the embedding medium removal process to a labeled antibody; and a label detecting process of detecting the labeled antibody bound to the cell membrane protein after the labeling process.

[1-3] The method according to [1-2], comprising an antigen retrieval process of retrieving the cell membrane protein in the fixed tissue after the embedding medium removal process.

[1-4] The method according to [1-2] or [1-3], wherein the labeling process comprises a primary antibody reaction step of reacting a primary antibody having immunological reactivity against the cell membrane protein with the cell membrane protein of the fixed tissue after the embedding medium removal process, and a secondary antibody reaction step of reacting a labeled secondary antibody having immunological reactivity against the primary antibody with the fixed tissue after the primary antibody reaction step.

[2-1] The method according to any one of [1-1] to [1-4], wherein the embedding medium removal process is a process of removing the embedding medium by contacting, without baking, the fixed tissue with the removal solution.

[2-2] The method according to any one of [1-1] to [2-1], wherein the embedding medium removal process is a process of removing the embedding medium by contacting the fixed tissue with the removal solution at a temperature less than the melting point of the embedding medium.

[3] The method according to any one of [1-1] to [2-2], wherein the removal solution is a solution comprising a surfactant and/or an organic solvent.

[4] The method according to any one of [1] to [3], wherein the contact in the embedding medium removal process is immersion.

[5] The method according to any one of [1] to [4], wherein the antigen retrieval process comprises a heat treatment step at 90 to 130°C.

[6] The method according to any one of [1] to [5], comprising a cooling process of cooling the fixed tissue after the antigen retrieval process.

[7] The method according to any one of [1] to [6], wherein the labeled secondary antibody is a complex of an antibody having immunological reactivity against the primary antibody and peroxidase bound onto a polymer carrier.

[8] The method according to [7], wherein the label detecting process is a process of detecting the cell membrane protein colored by a coloring reagent reactive with the peroxidase.

[9] The method according to [8], wherein the coloring reagent is 3,3'-diaminobenzidine (DAB).

[10-1] The method according to any one of [1] to [9], wherein the embedding medium is paraffin.

[10-2] The method according to [10-1], wherein the embedding medium removal process is a process of removing the embedding medium by contacting the fixed tissue with the removal solution while maintaining the fixed tissue at less than 45°C.

[11-1] The method according to any one of [1] to [10-2], wherein the cell membrane protein is a cell membrane protein expressed on the surface of a cancer cell.

[11-2] The method according to any one of [1] to [11-1], wherein the cell membrane protein is a disease marker.

[12] The method according to [11-2], wherein the disease is cancer.

[13] The method according to [11-2] or [12], wherein the disease marker is CAPRIN-1 protein.

[14] A method for determining a treatment for an individual from whom a fixed tissue is derived, comprising: a process of detecting disease markers in the embedded fixed tissue using a method according to any one of [11] to [13]; and a treatment determining process of determining a treatment for the individual based on the disease markers detected by the process.

[15] An apparatus for detecting a cell membrane protein in an embedded fixed tissue, comprising: an embedding medium removal unit of removing the embedding medium by contacting the fixed tissue comprising an embedding medium in a solid state with a removal solution; a labeling reaction unit of binding the cell membrane protein of the fixed tissue that has been subjected to the embedding medium removal unit to a labeled antibody; and a label detecting unit of detecting the labeled antibody bound to the cell membrane protein in the fixed tissue that has been subjected to the labeling reaction unit.

[16] The apparatus according to [15], wherein the embedding medium removal unit is equipped with a temperature regulating means for making the fixed tissue less than the melting point of the embedding medium.

[17] The apparatus according to [15] or [16], further comprising an antigen retrieval unit of retrieving the cell membrane protein in the fixed tissue that has been subjected to the embedding medium removal unit.

[18] The apparatus according to any one of [15] to [17], wherein the cell membrane protein is a cell membrane protein expressed on the surface of a cancer cell.

[19] The apparatus according to any one of [15] to [18], wherein the cell membrane protein is a disease marker.

[20] The apparatus according to [19], wherein the disease is cancer.

[21] The apparatus according to [19] or [20], wherein the disease marker is CAPRIN-1 protein.

The present description includes the disclosure of Japanese Patent Application Number 2023-050776 as the basis of the priority of the present application.

Advantageous Effects of Invention

According to the detection method of the present invention, a cell membrane protein can be detected with high accuracy in an embedded fixed tissue.

According to the treatment determination method of the present invention, an appropriate treatment can be determined based on expression information of a cell membrane protein detected with high accuracy.

Brief Description of Drawings

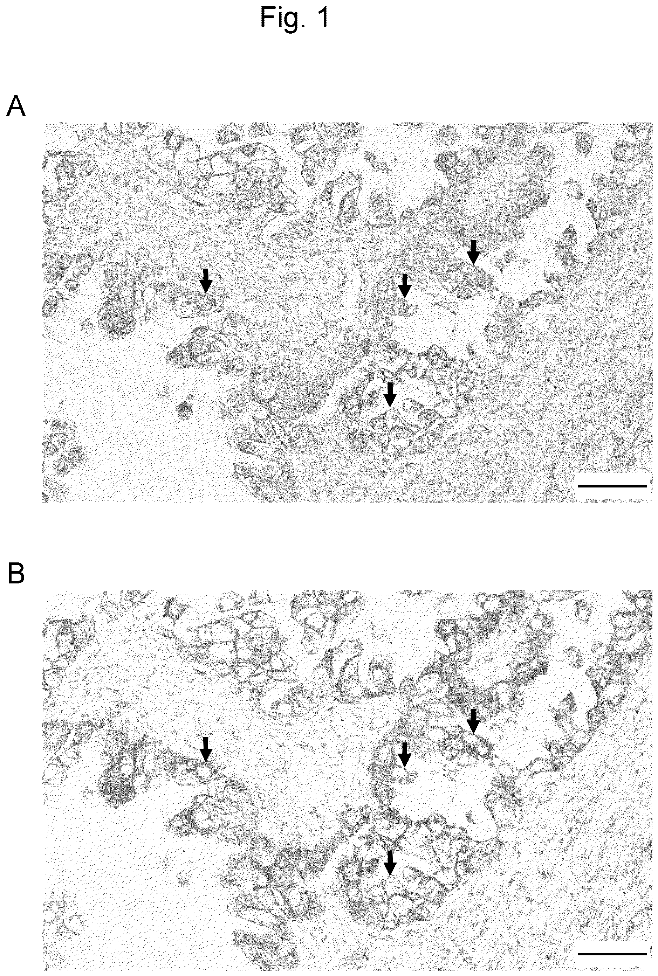

[Figure 1] Figure 1 shows exemplary staining images of an ovarian cancer tissue, obtained using the staining method of Example 1. Panel A shows a staining image of cell nuclei and CAPRIN-1 protein. Panel B shows a staining image obtained by excluding the signals of the cell nuclei. In the figure, each arrow indicates a cell whose cell membrane was appropriately stained, and each scale bar represents 50 $\mu$m.

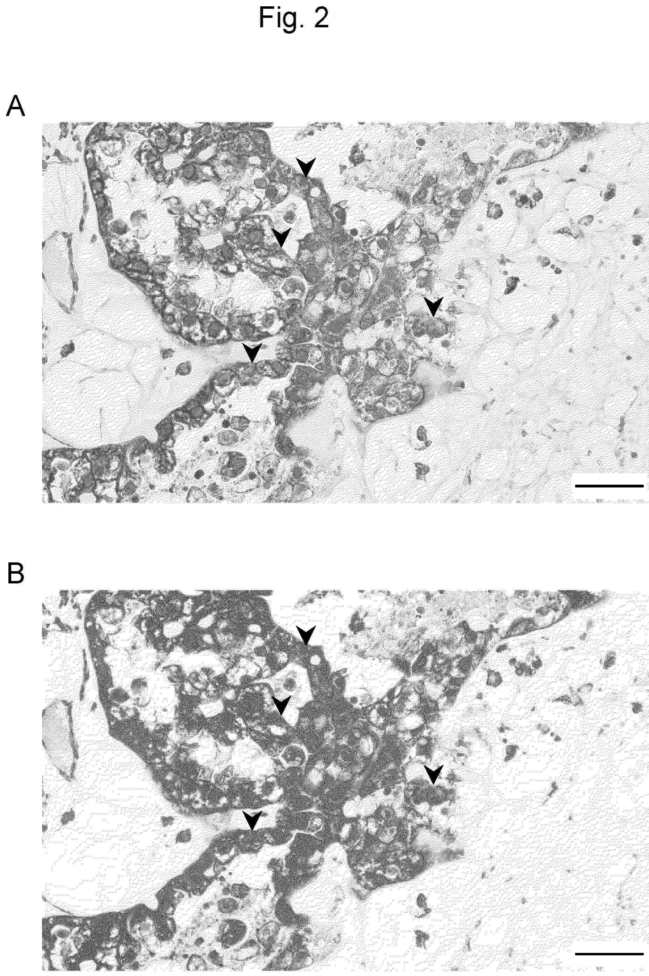

[Figure 2] Figure 2 shows exemplary staining images of an ovarian cancer tissue, obtained using the staining method of Comparative Example 1. Panel A shows a staining image of cell nuclei and CAPRIN-1 protein. Panel B shows a staining image obtained by excluding the signals of the cell nuclei. In the figure, each arrowhead indicates an inappropriately stained cell whose cytoplasm was also stained, and each scale bar represents 50 $\mu$m.

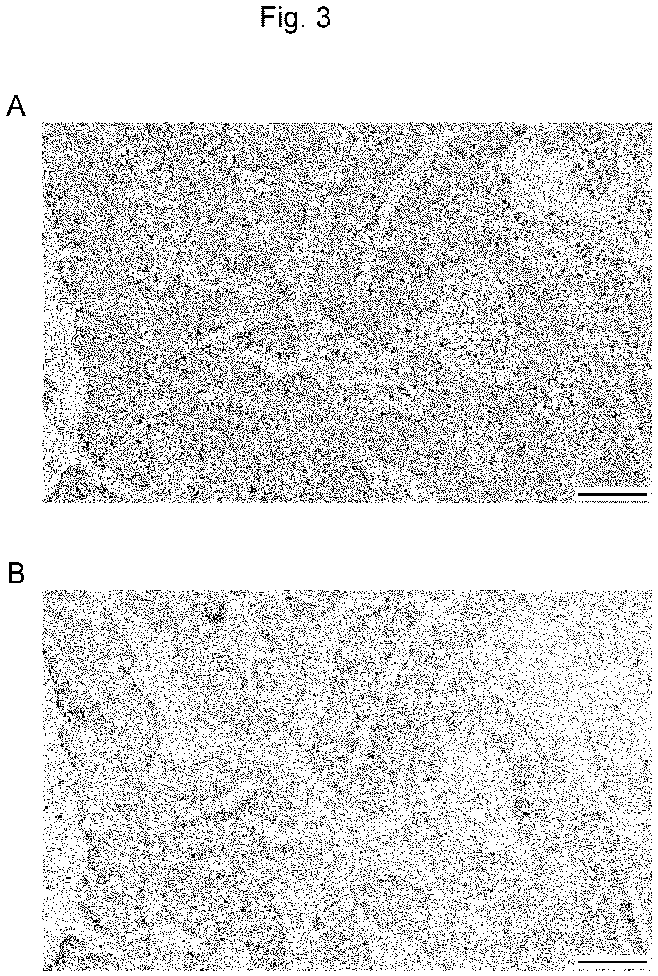

[Figure 3] Figure 3 shows staining images of a gastric cancer tissue that was scored as 0 (CAPRIN-1 negative). Panel A shows a staining image of cell nuclei and CAPRIN-1 protein. Panel B shows a staining image obtained by excluding the signals of the cell nuclei. In the figure, each scale bar represents 50 $\mu$m.

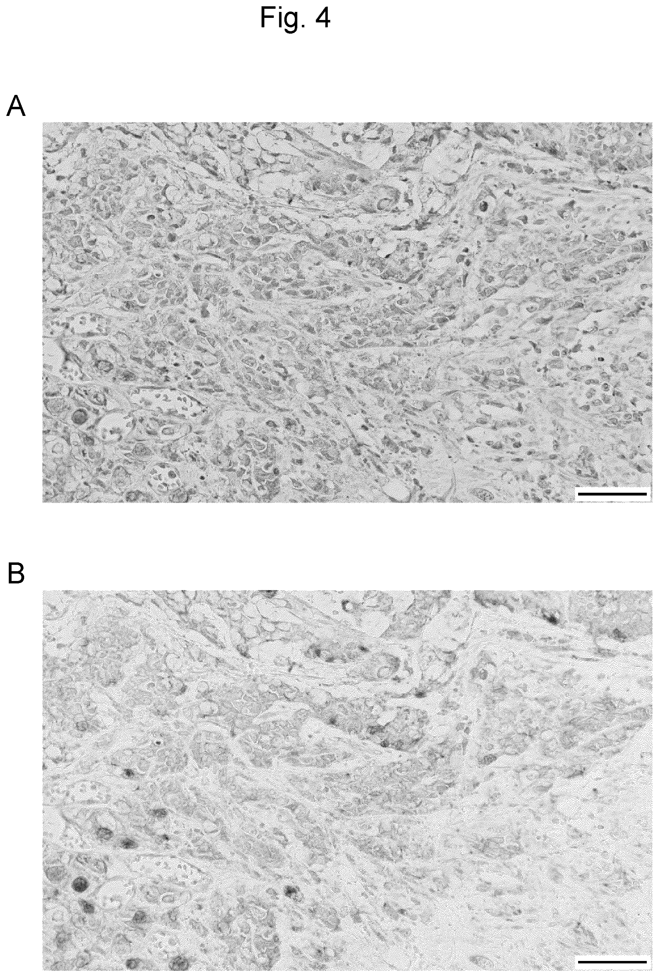

[Figure 4] Figure 4 shows staining images of a gastric cancer tissue that was scored as 1 (CAPRIN-1 negative). Panel A shows a staining image of cell nuclei and CAPRIN-1 protein. Panel B shows a staining image obtained by excluding the signals of the cell nuclei. In the figure, each scale bar represents 50 $\mu$m.

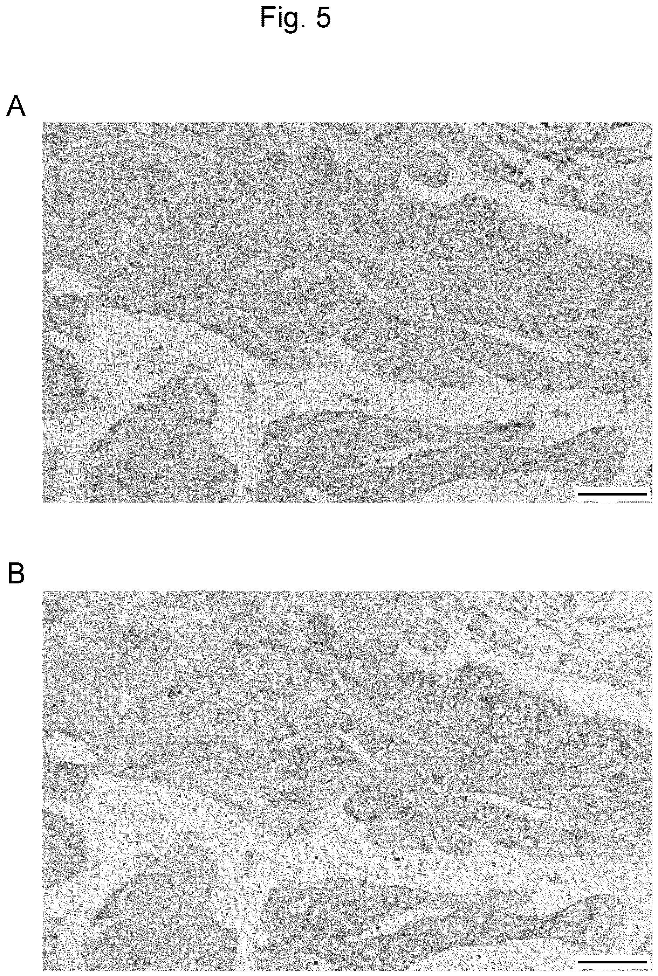

[Figure 5] Figure 5 shows staining images of a gastric cancer tissue that was scored as 2 (CAPRIN-1 positive). Panel A shows a staining image of cell nuclei and CAPRIN-1 protein. Panel B shows a staining image obtained by excluding the signals of the cell nuclei. In the figure, each scale bar represents 50 $\mu$m.

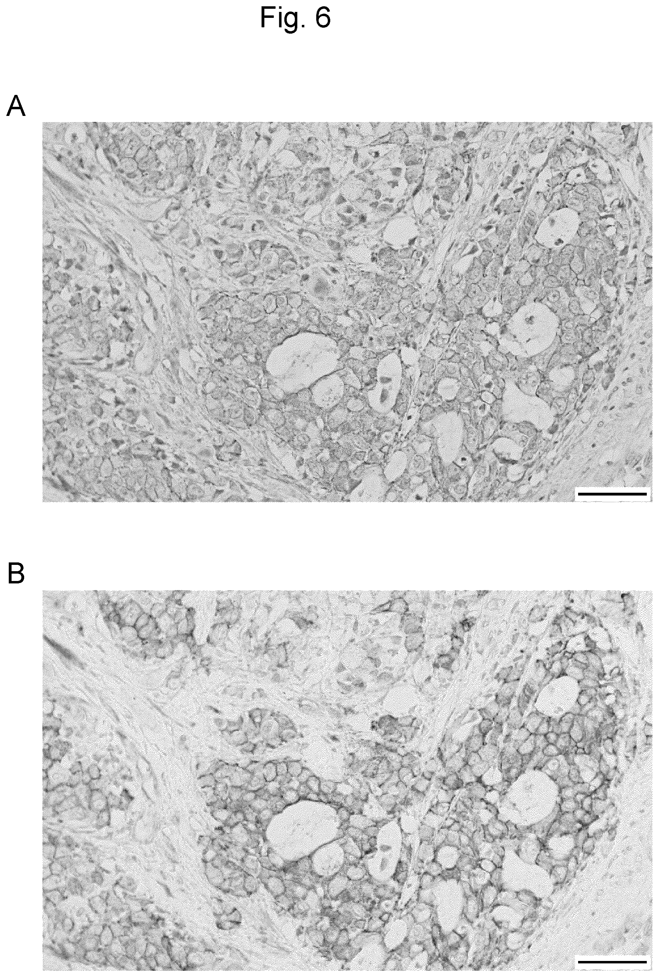

[Figure 6] Figure 6 shows staining images of a gastric cancer tissue that was scored as 3 (CAPRIN-1 positive). Panel A shows a staining image of cell nuclei and CAPRIN-1 protein. Panel B shows a staining image obtained by excluding the signals of the cell nuclei. In the figure, each scale bar represents 50 $\mu$m.

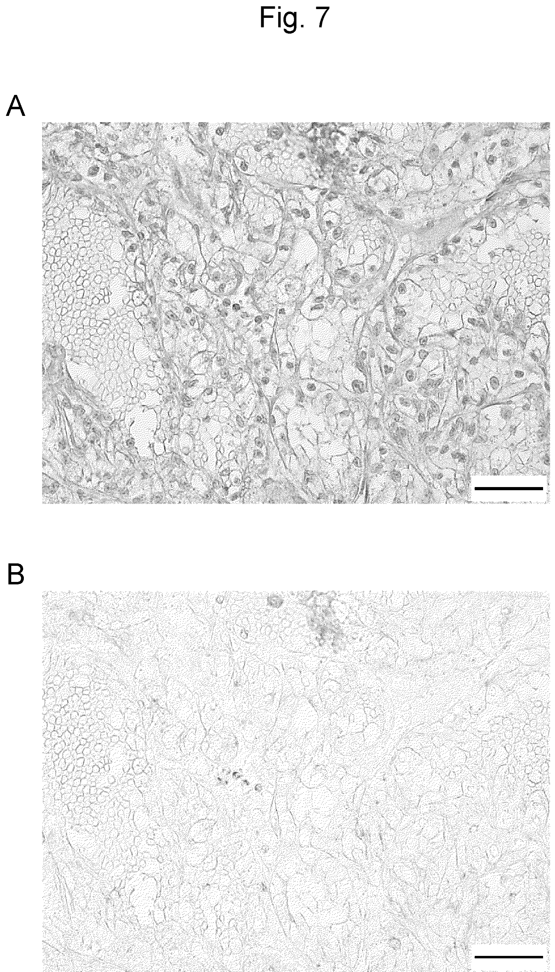

[Figure 7] Figure 7 shows staining images of a renal cancer tissue that was scored as 0 (CAPRIN-1 negative). Panel A shows a staining image of cell nuclei and CAPRIN-1 protein. Panel B shows a staining image obtained by excluding the signals of the cell nuclei. In the figure, each scale bar represents 50 $\mu$m.

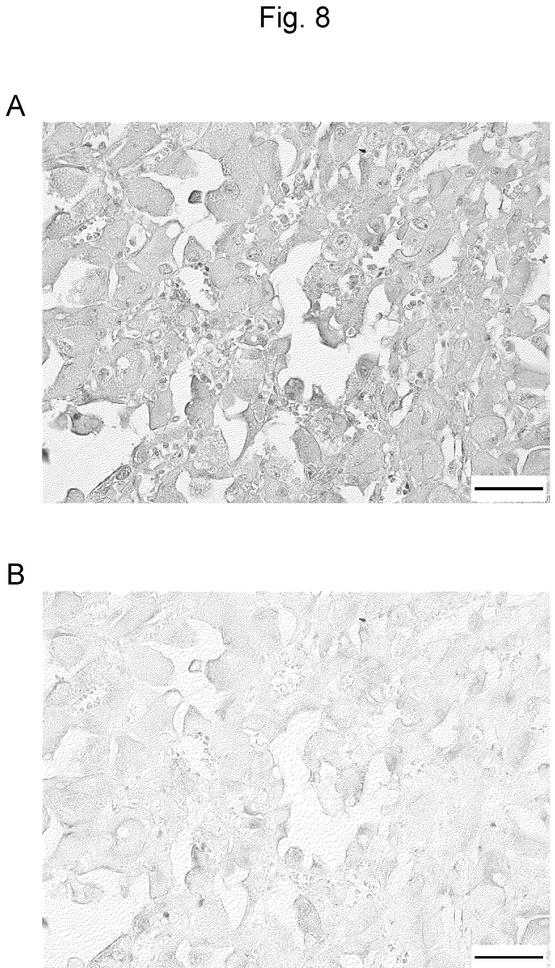

[Figure 8] Figure 8 shows staining images of a renal cancer tissue that was scored as 1 (CAPRIN-1 negative). Panel A shows a staining image of cell nuclei and CAPRIN-1 protein. Panel B shows a staining image obtained by excluding the signals of the cell nuclei. In the figure, each scale bar represents 50 $\mu$m.

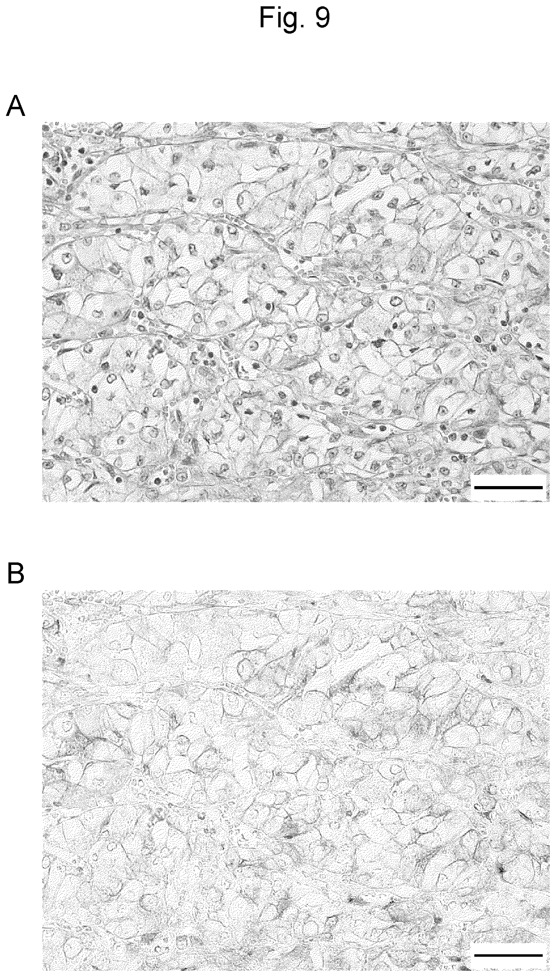

[Figure 9] Figure 9 shows staining images of a renal cancer tissue that was scored as 2 (CAPRIN-1 positive). Panel A shows a staining image of cell nuclei and CAPRIN-1 protein. Panel B shows a staining image obtained by excluding the signals of the cell nuclei. In the figure, each scale bar represents 50 $\mu$m.

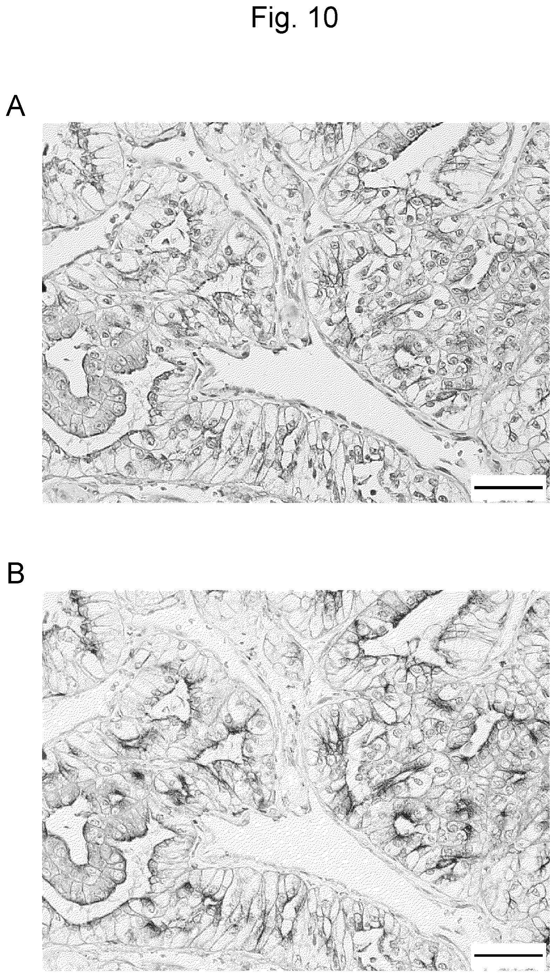

[Figure 10] Figure 10 shows staining images of a renal cancer tissue that was scored as 3 (CAPRIN-1 positive). Panel A shows a staining image of cell nuclei and CAPRIN-1 protein. Panel B shows a staining image obtained by excluding the signals of the cell nuclei. In the figure, each scale bar represents 50 $\mu$m.

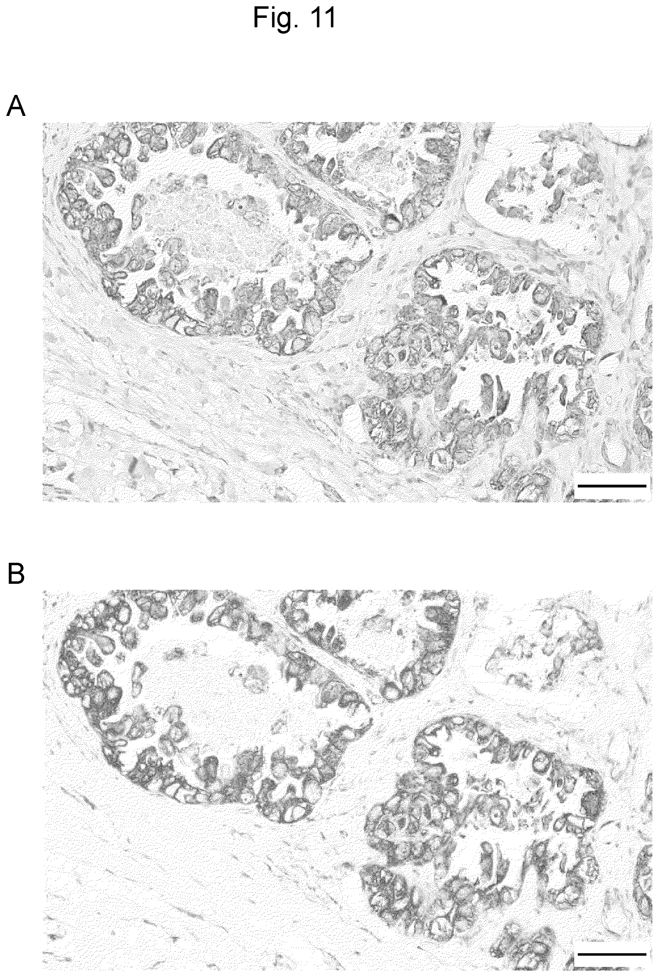

[Figure 11] Figure 11 shows exemplary staining images of an ovarian cancer tissue, obtained using the staining method of Example 1. Panel A shows a staining image of cell nuclei and CAPRIN-1 protein. Panel B shows a staining image obtained by excluding the signals of the cell nuclei. In the figure, each scale bar represents 50 $\mu$m.

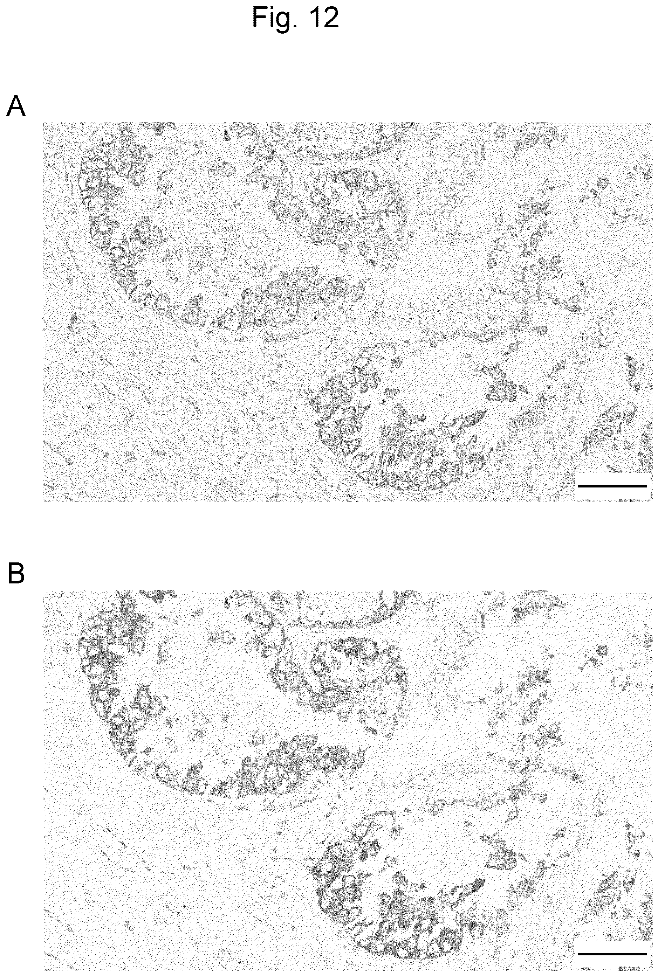

[Figure 12] Figure 12 shows exemplary staining images of an ovarian cancer tissue, obtained using the staining method of Example 3. Panel A shows a staining image of cell nuclei and CAPRIN-1 protein. Panel B shows a staining image obtained by excluding the signals of the cell nuclei. In the figure, each scale bar represents 50 $\mu$m.

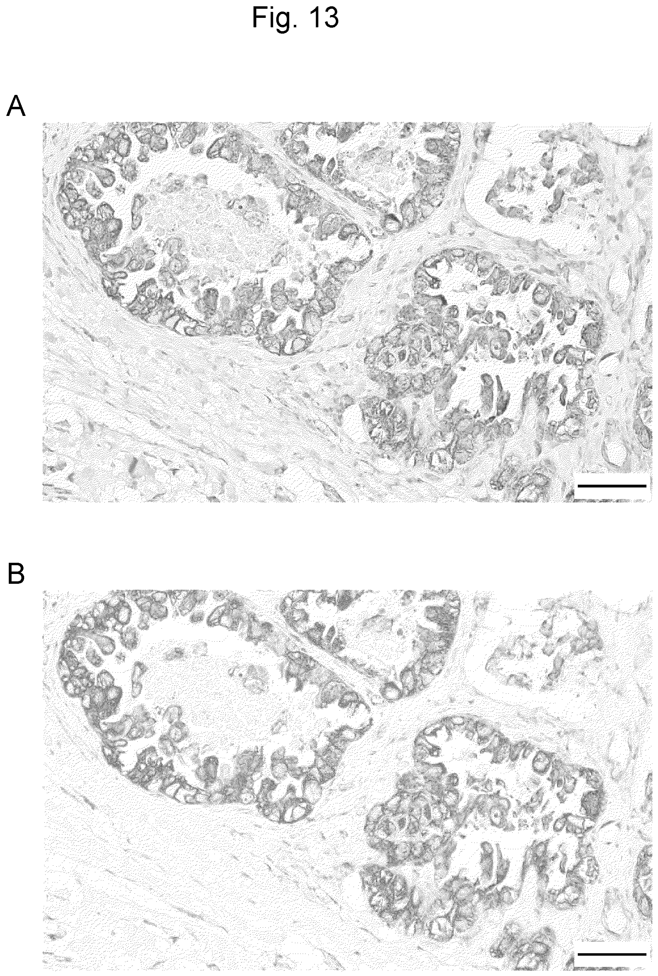

[Figure 13] Figure 13 shows exemplary staining images of an ovarian cancer tissue, obtained using the staining method of Example 4. Panel A shows a staining image of cell nuclei and CAPRIN-1 protein. Panel B shows a staining image obtained by excluding the signals of the cell nuclei. In the figure, each scale bar represents 50 $\mu$m.

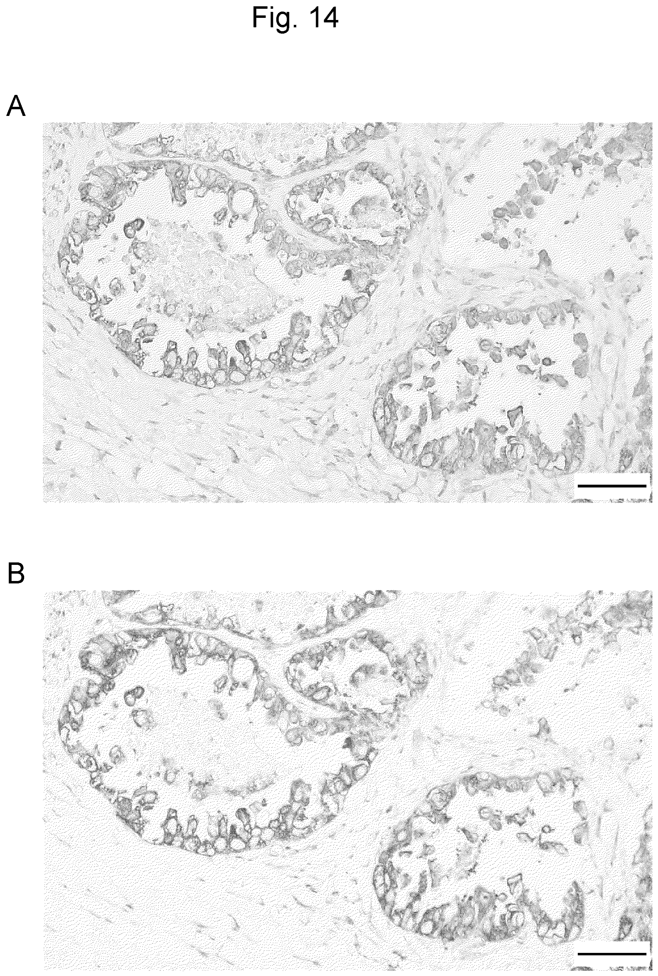

[Figure 14] Figure 14 shows exemplary staining images of an ovarian cancer tissue, obtained using the staining method of Example 5. Panel A shows a staining image of cell nuclei and CAPRIN-1 protein. Panel B shows a staining image obtained by excluding the signals of the cell nuclei. In the figure, each scale bar represents 50 $\mu$m.

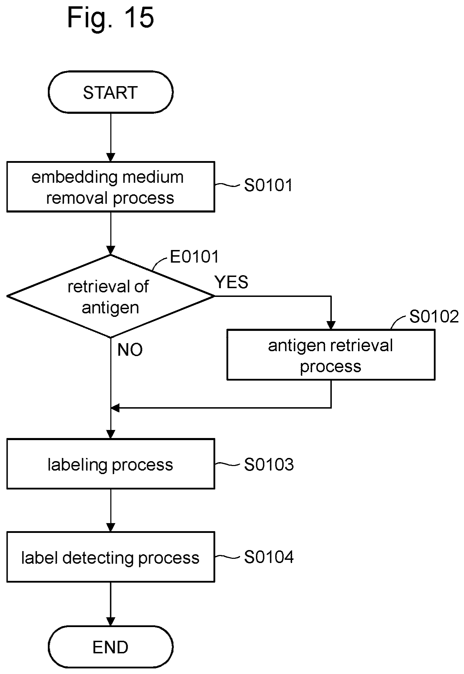

[Figure 15] Figure 15 is a flow chart showing an exemplary embodiment of a method for detecting a cell membrane protein.

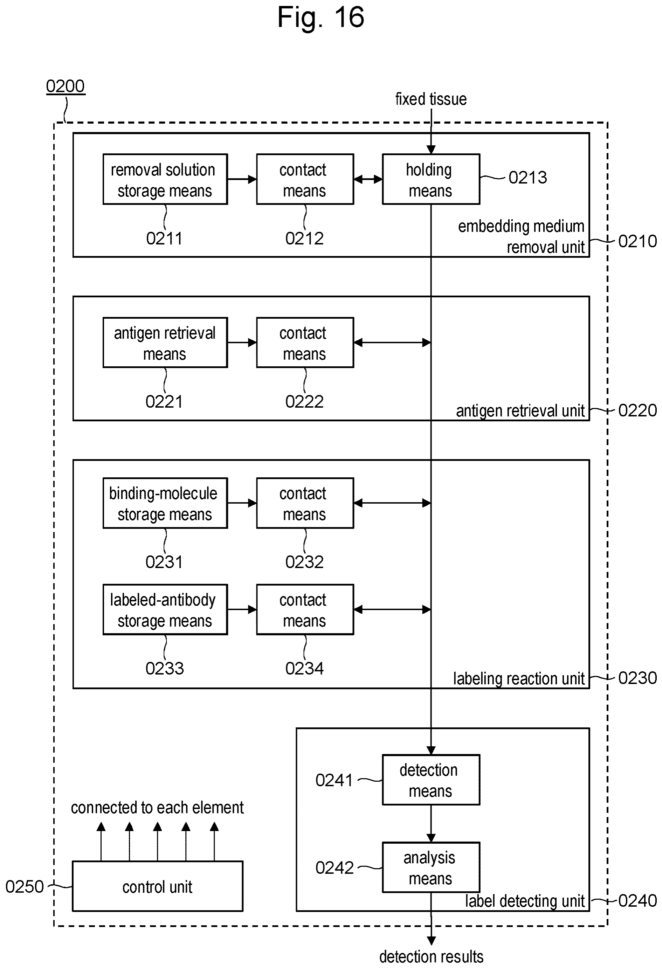

[Figure 16] Figure 16 is an exemplary functional block diagram of an apparatus for detecting a cell membrane protein according to a third aspect.

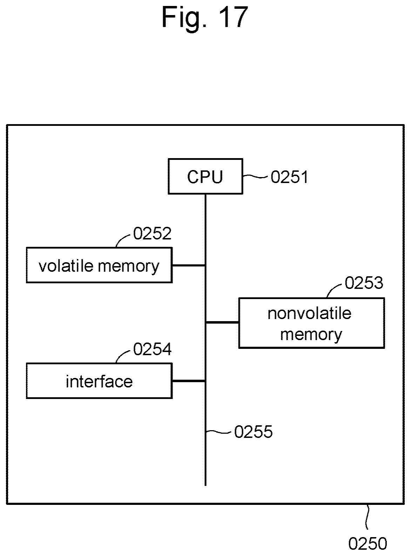

[Figure 17] Figure 17 is a diagram showing an exemplary hardware configuration of a control unit according to the apparatus for detecting a cell membrane protein.

Description of Embodiments

1. Method for Detecting Cell Membrane Protein

1-1. Summary

A first aspect of the present invention is a method for detecting a cell membrane protein. The method of the present aspect comprises an embedding medium removal process, a labeling process, and a label detecting process as essential processes, and comprises an antigen retrieval process, a cooling process, a nonspecific reaction suppressing process, and a coloring process as optional processes. According to the method of the present aspect, a cell membrane protein can be detected with high specificity.

1-2. Definitions

The term "embedding medium" refers to a reagent used for embedding. The term "embedding" refers to the process of infiltrating a reagent into a biological sample to harden the biological sample.

The term "fixed tissue" refers to a biological sample comprising a tissue that has been fixed. The term "fixation" refers to a treatment in which the proteins in a biological sample are denatured and/or precipitated, thereby protecting the biological sample from degradation due to autolysis or putrefaction. The fixation herein includes any fixation treatment utilizing a cross-linking agent, a coagulation-precipitation agent, or a combination thereof.

The term "biological sample" refers to a sample composed of cells of an organism. In particular, the "biological sample" herein includes both a sample isolated from an organism individual and a sample comprising a cell prepared in vitro. The organism individual herein is not particularly limited as long as it is a eukaryotic individual, and includes, for example, individuals of mammals such as primates, pet animals, domestic animals, and sport animals. Further, preferred organisms include individuals of humans, horses, pigs, cows, sheep, goats, dogs, and cats.

The term "cell membrane protein" refers to a protein that is present on the cell membrane. The "cell membrane protein" in the present invention includes any of a transmembrane protein, a peripheral cell membrane protein (cell membrane superficial protein), and a lipid-modified protein. Both the peripheral cell membrane protein and the lipid-modified protein are proteins without a transmembrane domain.

The "transmembrane domain" is a protein domain having affinity to the lipid bilayer constituting the cell membrane, and penetrating the lipid bilayer. On the other hand, in the cell membrane protein, the protein domain exposed to the outside of the cell is called the extracellular domain, and the protein domain exposed to the inside of the cell is called the intracellular domain. For example, some lipid-modified proteins and peripheral cell membrane proteins are present without being embedded in the cell membrane at all, and hence the entire molecule of each of such proteins can be said to be an extracellular domain or an intracellular domain.

The term "solid state" refers to a state without fluidity. The solid state herein preferably does not include a semi-solid state, such as the gel-like state.

The term "having immunological reactivity" herein means a property by which an antibody binds to an antigen or a partial polypeptide thereof in vivo. For example, it refers to having an activity to bind to an antigen based on antigen-antibody reaction. Said binding to the antigen includes both specific binding and nonspecific binding.

The term "antibody" refers to an immunoglobulin or an antigen-binding fragment thereof that specifically binds to another molecule. The "antibody" herein includes any of a monoclonal antibody and a polyclonal antibody, and an antigen-binding fragment thereof.

The term "label" refers to a substance whose presence is detectable. The term "labeling" of a substance herein refers to linking the substance to a label by covalent bonding.

The term "baking" refers to the treatment of heating in the atmosphere. The term "baking" herein refers especially to the treatment of heating a fixed tissue in the atmosphere to a temperature of the melting point of an embedding medium or more. This treatment is usually carried out to partially melt the embedding medium, and to adhere the tissue to a glass slide or the like.

The term "disease marker" refers to a biomolecule (such as a nucleic acid molecule or a protein molecule) that serves as an indicator to determine the development of a disease or the presence or absence of the risk of onset of a disease. The term herein refers especially to a protein that serves as an indicator to determine the development of a disease or the presence or absence of the risk of onset of a disease.

The term "cancer" means a malignant neoplasm, and is used interchangeably with the term "tumor". The term "cancer" herein includes any of a primary cancer, a metastatic cancer, a cancer that has metastasized, and a cancer that has recurred.

The term "CAPRIN-1 (cytopasmic-activation and proliferation-associated protein 1) protein" refers to a cell membrane protein expressed on the cell membrane mainly in cancer cells. The CAPRIN-1 protein herein also includes a protein having a biological function equivalent to that of human CAPRIN-1 protein, such as a congener (that is, a homolog or an ortholog), a variant due to genetic polymorphism, and a derivative.

The term "treatment" refers to a treatment for completely or partially curing or ameliorating, or for preventing or delaying the progression of, a disease or condition, or a symptom associated therewith, in an individual already having the symptom. The term "prophylaxis" means a treatment for preventing or delaying the onset of a disease or condition in an individual who may develop the disease or condition. The term "therapeutic method" or "prophylactic method" refers to a method used for a therapeutic or prophylactic procedure.

1-3. Method

The method of the present aspect comprises an embedding medium removal process, a labeling process, and a label detecting process as essential processes, and comprises an antigen retrieval process, a cooling process, a nonspecific reaction suppressing process, and a coloring process as optional processes. Each process is described below in detail.

1-3-1. Embedding Medium Removal Process (S0101)

The "embedding medium removal process (S0101)" is an essential process, and is a process of contacting a fixed tissue comprising an embedding medium in a solid state with a removal solution, to remove the embedding medium.

<Embedding Medium>

The embedding medium is not particularly limited as long as it is a medium capable of embedding and hardening the tissue. The embedding medium may be appropriately selected according to, for example, the type of the subject fixed tissue, the temperature condition for the embedding, and the detection method used in the label detecting process.

For example, when a temperature of more than 0°C is employed in the embedding, the embedding medium specifically includes, for example, waxes, paraffin (such as Paraplast, Broloid, and Tissuecan), paraffin waxes, acrylic resins, methacrylate resins (such as ethylene glycol dimethacrylate, glycol methacrylate, butyl methacrylate, hydroxypropyl methacrylate, and methyl methacrylate), dammar resins, epoxy resins, divinylbenzene, and other plastic resins (such as Spar Plastic, Lowicryl (registered trademark), Epon, Araldite, LR White, and Durcupan), and copolymers of combinations thereof. Paraffin and paraffin waxes are preferred as the embedding medium herein.

Specifically, when a temperature of 0°C or less is employed in the embedding, the embedding medium includes, for example, Optimum Cutting Temperature (OCT) compounds (such as the Tissue-Tek (registered trademark) O. C. T. compound or the Tissue-plus (registered trademark) O. C. T. compound), the PELCO (registered trademark) freeze-embedding compound, PolarStat (trademark), the PolarStat Plus (trademark) embedding medium, and the Tissue Freezing Medium (TFM (trademark)). Each of these embedding media may comprise water-soluble glycol and a resin. Specifically, the embedding medium comprises, for example, 5 to 15% polyvinyl alcohol and 1 to 10% polyethylene glycol.

The embedding medium may be diluted with an organic solvent or an aqueous solution as appropriate to achieve a desired hardness.

<Fixed Tissue>

The fixed tissue used in this process is a biological sample from which the cell membrane protein is to be detected by the method of the present aspect.

The individual, organ, or tissue from which the fixed tissue is derived is not particularly limited. For example, the individual may be any of a healthy individual, an individual who may have developed a disease, and an individual who has developed a disease.

The term "healthy individual" herein refers to an individual in a healthy state. The term "healthy state" herein means at least a state free from the development of the disease to be examined, preferably a healthy state free from any disease or disorder.

The fixed tissue may comprise a diseased tissue, may potentially comprise a diseased tissue, or may comprise only a normal tissue.

In the method of the present aspect, a fixed tissue derived from a single individual may be used as the fixed tissue, or fixed tissues derived from a plurality of individuals may be used at the same time. The fixed tissue derived from each individual may be derived from a single organ, or may be derived from a plurality of organs.

The type of the disease is not particularly limited. The type of the disease includes, for example, cancers and inflammations, and the tissue from which the fixed tissue is derived includes, for example, cancer tissues and inflammatory tissues. The cell membrane protein detected by the method of the present aspect may be, for example, a cell membrane protein expressed on the surface of a diseased cell, and such a cell membrane protein includes, for example, a cell membrane protein expressed on the surface of an inflammatory cell, and a cell membrane protein expressed on the surface of a cancer cell.

The cancer to be targeted in the present invention is not particularly limited. For example, the cancer may be a cancer showing expression of CAPRIN-1 protein on the surface of the cell membrane. The cancer preferably includes, for example, basal cell cancer, Paget's disease, skin cancer, breast cancer, renal cancer, pancreatic cancer, colon cancer, lung cancer, brain tumor, gastric cancer, uterus cancer, ovarian cancer, prostate cancer, urinary bladder cancer, esophageal cancer, leukemia, lymphoma, liver cancer, gallbladder cancer, sarcoma, mastocytoma, adrenal cortex cancer, Ewing's tumor, Hodgkin's lymphoma, mesothelioma, multiple myeloma, testicle cancer, thyroid cancer, head and neck cancer, Bowen's disease, melanoma, prickle cell carcinoma, extramammary Paget's disease, mycosis fungoides, Sezary's syndrome, cutaneous T/NK-cell lymphoma, T-cell leukemia or lymphoma having a lesion only in the skin, cutaneous B-cell lymphoma (indolent group), cutaneous T-cell lymph mammary adenoma, complex mammary adenoma, malignant mixed tumor of mammary gland, intraductal papillary adenocarcinoma, lung adenocarcinoma, squamous cell carcinoma, small cell carcinoma, large cell carcinoma, glioma which is a neuroepithelial tissue tumor, glioblastoma, neuroblastoma, ependymoma, neuronal tumor, embryonal neuroectodermal tumor, neurilemoma, neurofibromatosis, meningioma, chronic lymphocytic leukemia, alimentary lymphoma, gastrointestinal lymphoma, small to medium cell lymphoma, cecal cancer, ascending colon cancer, descending colon cancer, transverse colon cancer, sigmoid colon cancer, rectal cancer, ovarian epithelial cancer, germ cell tumor, interstitial cell tumor, pancreatic ductal cancer, invasive pancreatic ductal cancer, adenocarcinoma of pancreatic cancer, acinar cell carcinoma, adenosquamous cancer, giant cell tumor, intraductal papillary mucinous tumor, mucinous cystadenocarcinoma, pancreatoblastoma, pancreatic islet cell tumor, Frantz tumor, serous cystadenocarcinoma, solid papillary carcinoma, gastrinoma, glucagonoma, insulinoma, multiple endocrine neoplasia type-1 (Wermer syndrome), nonfunctional islet cell tumor, somatostatinoma, VIP secreting tumor, uterine cervical cancer, uterine body cancer, fibrosarcoma, osteo-/joint sarcoma, Ewing's sarcoma, Wilms' tumor, hepatoblastoma, soft tissue sarcoma, acute leukemia, chronic leukemia, spinal cord tumor, malignant soft tissue tumor, and tumors of the teratoma group, wherein the head and neck cancer includes, for example, hypopharynx cancer, oropharynx cancer, tongue cancer, nasopharyngeal carcinoma, oral cavity cancer, lip cancer, sinus cancer, and laryngeal cancer, but is not particularly limited thereto. The cancer also includes those originating from the cancers described above, including palpable cancers, cancers that are subcutaneously located, cancers that are intradermally located, superficial cancers, cancers that are located in the dermis, and cancers located in a non-parenchymal organ.

The type of cell membrane protein detected by the method of the present aspect is not particularly limited. The cell membrane protein may be any of a transmembrane protein, a peripheral cell membrane protein, and a lipid-modified protein, or may be a protein localized on the cell membrane under a specific condition. For example, the cell membrane protein may be a cell membrane protein without a transmembrane domain, or may be a cell membrane protein that is fully or partially exposed to the outside of the cell.

The cell membrane protein herein includes, for example, disease markers such as cancer markers. Specifically, the cancer markers include, for example, proteins such as CAPRIN-1, HER2, CD20, carcinoembryonic antigen (CEA), CA-125, CA19-9, CD117, ALK, BCR-ABL1, BRAF, CFTR, EGFR, IL2RA, RAS, and Claudin 8.

The fixation method for the fixed tissue is not particularly limited, and a method known in the art may be used. For example, a method described in Hopwood D. "Fixatives and fixation: a review." Histochem J. 1969; 1(4): 323-60, or a method described in "Immunohistochemical Staining Methods" 6th ed. (2013) (DAKO North America, Inc.) may be used. The reagent used for the fixation is not particularly limited. The reagent may be, for example, a cross-linking agent, a coagulation-precipitation agent, or a combination thereof. Specifically, the fixing reagent includes, for example, cross-linking agents such as solutions comprising glutaraldehyde, formaldehyde, paraformaldehyde, paraformaldehyde-picric acid, periodate-lysine-paraformaldehyde, or zinc ion-formaldehyde; coagulation-precipitation agents such as methanol, ethanol, acetone, acetic acid-zinc chloride, and methanol-acetone mixtures; and mixtures thereof (such as formalin solutions). When necessary, the fixing reagent may be diluted with a buffer or the like before use.

The fixation conditions are not particularly limited. For example, the fixation temperature includes 0°C or less, 4°C to 45°C, 4°C to 37°C, 4°C to 30°C, and 4°C to 25°C. Further, the fixation time is not particularly limited. For example, the fixation time includes 30 seconds to 48 hours, 10 minutes to 36 hours, 20 minutes to 24 hours, and 30 minutes to 20 hours. The number of times of the fixation is not particularly limited. For example, the fixation may be carried out once or more, twice or more, or 3 times or more. When the fixation is carried out a plurality of times, the method, the reagent used, and the conditions may be the same or different among the times of the fixation.

When necessary, the fixation may be followed by dehydration treatment or replacement treatment. The conditions for each treatment are not particularly limited, and any method known in the art may be employed therefor. For example, the method described for the washing step in the embedding medium removal process may be used.

The fixed tissue used in the method of the present aspect may be a fixed tissue in the form of a tissue piece embedded in the above-described embedding medium, or may be a section thereof. The fixed tissue is preferably a section.

The method of preparing the section is not particularly limited. The section may be prepared using a method known in the art, for example, by manual sectioning or by using a slicer (such as a microtome, a vibratome, or a cryostat).

The thickness of the section is not particularly limited. Specifically, the thickness includes, for example, a thickness of 0.5 μm or more, 1 μm or more, 2 μm or more, 3 μm or more, or 4 μm or more. Further, the thickness may be, for example, 300 μm or less, 200 μm or less, 150 μm or less, 100 μm or less, 80 μm or less, 60 μm or less, 50 μm or less, 30 μm or less, 20 μm or less, 15 μm or less, 10 μm or less, 8 μm or less, 7 μm or less, 6 μm or less, or 5 μm or less. The thickness of the section may be, for example, within the range of 1 μm to 300 μm, 1 μm to 100 μm, 1 μm to 50 μm, 1 μm to 20 μm, 1 μm to 10 μm, 2 μm to 100 μm, 2 μm to 50 μm, 2 μm to 20 μm, or 2 μm to 10 μm.

The section may be placed on a support such as a glass slide, or may be, for example, floating in a solution without being placed on a support. Preferably, the section is placed on a support such as a glass slide. The material of the support is not particularly limited, and the support may comprise, for example, a polymer material, a glass material, a plastic material, or a metallic material. The glass material includes, for example, soda-lime glass, borosilicate glass, crown glass, and combinations thereof. The shape of the support is not particularly limited. For example, membranes, microtiter plates, beads, filters, test strips, slides, cover glasses, and test tubes may be used as support. When necessary, the support may be subjected to surface treatment. The section may be adhered to the support. The method of the adhesion is not particularly limited, and may be, for example, a method using a matrix of silane, gelatin, poly-L-lysine, or the like.

The fixed tissue comprises an embedding medium in a solid state. The term "embedding medium in a solid state" refers to an embedding medium that is not melted. In other words, in the fixed tissue used in the method of the present aspect, all or part of the embedding medium is not melted.

The ratio of the embedding medium in a solid state to the entire embedding medium in the fixed tissue is not particularly limited. For example, the ratio may be 30% or more, 40% or more, 50% or more, 60% or more, 70% or more, 80% or more, 90% or more, 95% or more, or 100%.

Preferably, the fixed tissue used in the embedding medium removal process has not been baked, and has not been subjected to heating at the melting point of the embedding medium or more in the atmosphere.

The term "melting point" refers to the transition temperature at which a substance in a solid state melts into a liquid state upon heating.

The heating temperature herein varies depending on the type of the embedding medium used. When an embedding medium for frozen sections is used, its melting point varies among products, but, for example, since a common O.C.T. compound has a melting point of - 15°C to -5°C, the heating temperature upon its use corresponds to a temperature higher than this temperature. Specifically, the fixed tissue is preferably not exposed to an atmosphere at a temperature of -20°C or more, -15°C or more, -10°C or more, -5°C or more, or -0°C or more for a certain length of time or more. When other embedding media are used, their melting points vary among products, but, for example, since a common paraffin wax has a melting point of 45°C to 75°C, the heating temperature upon its use corresponds to, for example, a temperature higher than this temperature. The fixed tissue is preferably not exposed to an atmosphere at a temperature of 40°C or more, 45°C or more, 50°C or more, 55°C or more, 60°C or more, or 62°C or more for a certain length of time or more.

The length of time of exposure herein is not particularly limited as long as it is sufficient for allowing the embedding medium in the fixed tissue to begin to melt. For example, the fixed tissue is preferably not exposed to an atmosphere at a temperature of the melting point of the embedding medium or more for a length of time of 1 minute or more, 2 minutes or more, 3 minutes or more, 5 minutes or more, 6 minutes or more, 7 minutes or more, 8 minutes or more, 9 minutes or more, 10 minutes or more, 11 minutes or more, 12 minutes or more, 14 minutes or more, or 15 minutes or more. For example, the fixed tissue is preferably not exposed to an atmosphere at a temperature of 50°C or more for 5 minutes or more, an atmosphere at a temperature of 55°C or more for 10 minutes or more, or an atmosphere at a temperature of 60°C or more for 15 minutes or more.

<Removal Solution>

The term "removal solution" herein refers to a solution capable of dissolving the embedding medium. The type of the removal solution is not particularly limited. When the embedding medium is a lipophilic substance, for example, a solution comprising a surfactant and/or an organic solvent may be used. Preferably, a solution comprising an organic solvent is used. On the other hand, when the embedding medium is a hydrophilic substance, for example, water and/or an aqueous solution (such as an aqueous solution comprising a sugar such as sucrose; or a buffer) may be used.

The organic solvent used in this process is not particularly limited. For example, a linear, branched, or cyclic alkane having 4 to 16 carbon atoms (such as heptane or hexadecane); a linear, branched, or cyclic dialkyl ether having 4 to 16 carbon atoms (such as diethyl ether or dioctyl ether); an aromatic hydrocarbon such as toluene or xylene; an alcohol such as methanol, ethanol, or isopropyl alcohol; an alkyl halide such as chloroform; a monoterpene such as limonene; a vegetable oil such as citrus oil or coconut oil; or a mixture thereof; may be used. Alternatively, their substitutes (such as xylene substitutes), or removal solutions (such as Artisan Cleaning Solution (manufactured by Agilent)) attached to automated staining apparatuses may be used. Specifically, for example, the xylene substitutes include the Tissue Clear xylene substitute; the Shandon xylene substitute; aliphatic hydrocarbons such as heptane and hexadecane; derivatives of aliphatic hydrocarbons such as dioctyl ether; and mixtures thereof.

The concentration of the organic solvent contained in the removal solution is not particularly limited. The concentration may be, for example, 70% or more, 80% or more, 85% or more, 90% or more, 95% or more, 98% or more, 99% or more, or 100% in terms of the weight percent concentration. For example, xylene, and aqueous ethanol solutions at one or more concentrations may be used as the removal solution.

The removal solution used in this process is preferably xylene or a xylene substitute (such as a xylene substitute comprising an aliphatic hydrocarbon).

<Contact Step>

In this process, all or part of the embedding medium in the fixed tissue is dissolved into the removal solution by bringing the fixed tissue into contact with the removal solution.

The method of the contact is not particularly limited as long as it is a method for bringing the removal solution into contact with the embedding medium in the fixed tissue. For example, the removal solution may be sprayed, sprinkled, or applied to the fixed tissue, or the fixed tissue may be immersed in the removal solution. The contact in this process is preferably immersion. In the fixed tissue, the site of contact with the removal solution may be either all or part of the fixed tissue. However, when part of the fixed tissue is brought into contact with the removal solution, care should be taken to ensure that at least most of the embedding medium in the fixed tissue is brought into contact with the removal solution. For example, this process may be carried out such that 50% or more, 60% or more, 70% or more, 80% or more, 90% or more, 95% or more, or 100% of the embedding medium is brought into contact with the removal solution.

The conditions of the contact in this process are not particularly limited as long as the embedding medium can be dissolved into the removal solution.

For example, the fixed tissue may be brought into contact with the removal solution for a relatively long time at a relatively low temperature that is higher than the freezing point of the removal solution and lower than the melting point of the embedding medium.

The term "freezing point" refers to the transition temperature at which a substance in a liquid state solidifies into a solid state upon cooling.

The length of time in this case is not particularly limited, and the contact time may be, for example, 3 minutes or more, 5 minutes or more, 6 minutes or more, 8 minutes or more, 10 minutes or more, 11 minutes or more, 12 minutes or more, 13 minutes or more, 14 minutes or more, 15 minutes or more, 17 minutes or more, 19 minutes or more, or 20 minutes or more. The upper limit of the length of time is not particularly limited. For example, the fixed tissue may be brought into contact with the removal solution for a length of time of 48 hours or less, 36 hours or less, 30 hours or less, 24 hours or less, 20 hours or less, 18 hours or less, 16 hours or less, 15 hours or less, 12 hours or less, 8 hours or less, 6 hours or less, 3 hours or less, 2 hours or less, 90 minutes or less, 60 minutes or less, 30 minutes or less, 25 minutes or less, or 20 minutes or less.

The lower limit of the temperature in this case is not particularly limited as long as the temperature is higher than the freezing point of the removal solution. This temperature varies depending on the type of the removal solution used. For example, when xylene is used as the removal solution, the temperature may be a temperature higher than -25°C, which is the freezing point of xylene. When ethanol is used, the temperature may be higher than -114.5°C, which is the freezing point of ethanol. Specifically, the temperature may be, for example, -100°C or more, -50°C or more, -24°C or more, -10°C or more, 0°C or more, 1°C or more, 5°C or more, 10°C or more, 15°C or more, 20°C or more, or 25°C or more. The upper limit of the temperature in this case is not particularly limited as long as the temperature is lower than the melting point of the embedding medium. This temperature varies depending on the type of the embedding medium used. For example, when paraffin is used as the embedding medium, the temperature may be a temperature lower than 45°C to 75°C, which is the melting point of a paraffin wax. Specifically, the temperature may be, for example, less than 75°C, less than 70°C, less than 65°C, less than 60°C, less than 55°C, less than 50°C, less than 45°C, less than 40°C, less than 37°C, less than 35°C, less than 30°C, or less than 27°C. For example, the temperature may be room temperature (1 to 30°C) or normal temperature (15°C to 25°C). Specifically, for example, the fixed tissue may be brought into contact with an aromatic hydrocarbon such as xylene at normal temperature for 20 minutes or more.

The temperature may be maintained at a constant level during the contact. Alternatively, the temperature may be changed as appropriate, or may be changed naturally during the contact. For example, during the contact, the temperature is preferably maintained at a temperature lower than the melting point of the embedding medium. Specifically, for example, when paraffin is used as the embedding medium, the fixed tissue is preferably maintained at a temperature lower than the melting point of paraffin wax (for example, lower than 45°C).

The fixed tissue may be brought into contact with the removal solution a plurality of times. In this case, the removal solution and the contact conditions used may be the same or different among the times of the contact. For example, the removal solution may be renewed each time of the contact.

Further, for example, the fixed tissue may be brought into contact with the removal solution for a relatively short time at a relatively high temperature that is the melting point of the embedding medium or more, and less than the boiling point of the removal solution.

The term "boiling point" refers to the temperature at which the vapor pressure of a liquid is equal to the pressure of the system. The term "boiling point" herein refers especially to the temperature at which the vapor pressure of a liquid is equal to 1 atm.

The length of time in this case is not particularly limited, and may be, for example, 3 seconds or more, 5 seconds or more, 10 seconds or more, 15 seconds or more, 20 seconds or more, 25 seconds or more, or 30 seconds or more. The upper limit of the length of time is not particularly limited as long as an excessive burden is not caused on the fixed tissue. For example, the contact time may be 1 minute or less, 55 seconds or less, 50 seconds or less, 45 seconds or less, 40 seconds or less, 35 seconds or less, or 30 seconds or less.

The lower limit of the temperature in this case is not particularly limited as long as the temperature is the melting point of the embedding medium or more. This temperature varies depending on the type of the embedding medium used. For example, when paraffin is used as the embedding medium, the temperature may be the same as the melting point of the paraffin wax, that is, 45°C to 75°C, or a temperature higher than this. Specifically, the temperature may be, for example, 45°C or more, 50°C or more, 55°C or more, 60°C or more, 65°C or more, 70°C or more, 72°C or more, or 75°C or more. The upper limit of the temperature in this case is not particularly limited as long as the temperature is lower than the boiling point of the removal solution. For example, when xylene is used as the removal solution, the temperature may be a temperature lower than 138°C to 144°C, which is the boiling point of xylene. When ethanol is used, the temperature may be lower than 78.37°C, which is the boiling point of ethanol. Specifically, the temperature may be, for example, 140°C or less, 130°C or less, 120°C or less, 110°C or less, 100°C or less, 90°C or less, 85°C or less, 80°C or less, 78°C or less, or 75°C or less.

The proportion of the embedding medium removed by this process is not particularly limited as long as antigen-antibody reaction using a primary antibody or the like is not inhibited. For example, by this process, 50% or more, 60% or more, 70% or more, 80% or more, 90% or more, 95% or more, or 100% of the embedding medium is removed.

During this step, the removal solution may be allowed to flow as appropriate. In this case, the flow may be artificially generated by an operation such as stirring, or may be allowed to occur naturally due to convection or the like. Further, the fixed tissue may be moved as appropriate by, for example, changing the position of the fixed tissue.

<Separation Step>

When necessary, the contact with the removal solution may be followed by a separation step of separating the fixed tissue from the dissolved embedding medium.

The method of the separation is not particularly limited. Since the dissolved embedding medium is a liquid, and the fixed tissue is a solid, a solid-liquid separation method known in the art may be used. For example, the separation may be achieved by centrifugation using a dehydration apparatus or the like.

The separation step may be unnecessary, for example, when a large excess of the removal solution is used in the contact step, when the removal solution is allowed to flow, or when the removal solution is renewed.

<Washing Step>

When necessary, a washing step of washing away the removal solution and/or the like that has/have remained in the fixed tissue may be carried out. In the washing, a washing liquid is brought into contact with the fixed tissue. For the contact at this time, the method and conditions described for the contact step may be employed.

The washing liquid is not particularly limited as long as it is capable of removing the dissolved embedding medium remaining on the surface of the fixed tissue, the removal solution in which the embedding medium is dissolved, the removal solution in the fixed tissue, and/or the like. For example, among the solvents exemplified as the removal solution, a solvent that is different from the solvent used in the contact step may be used as the washing liquid. Preferably, when an organic solvent or a surfactant is used as the removal solution, a water-soluble solvent is used as the washing liquid. Specifically, for example, water, any buffer (such as phosphate buffer), alcohol (such as methanol or ethanol), or a mixture thereof may be used as the washing liquid. Preferably, an alcohol and/or a buffer, more specifically, ethanol and/or phosphate buffer may be used.

Washing may be performed a plurality of times. In this case, the washing liquid and the washing conditions used for each time of washing may be the same or different between washings. For example, when xylene is used as the removal solution, the fixed tissue may be washed with an alcohol such as ethanol, and then with a buffer such as phosphate buffer. Thus, in this process, xylene and ethanol may be used independently from each other. For example, the fixed tissue may be brought into contact with xylene, and then with ethanol. In this case, the concentration of the aqueous ethanol solution is not particularly limited. For example, one or more of the concentrations of 70% or more, 80% or more, 85% or more, 90% or more, 95% or more, 98% or more, 99% or more, or 100% in terms of the weight percent may be employed. For example, the fixed tissue may be brought into contact with ethanol solutions at 3 kinds of concentrations sequentially. Specifically, for example, the fixed tissue may be washed by being brought into contact with 100%, 90%, and 80% ethanol solutions in that order.

During this step, the washing liquid may be allowed to flow as appropriate. In this case, the flow may be artificially generated by an operation such as stirring, or may occur naturally due to convection or the like. Further, the fixed tissue may be moved as appropriate by, for example, changing the position of the fixed tissue.

The washing may also be performed in any other step.

1-3-2. Antigen Retrieval Process (S0102)

The "antigen retrieval process (S0102)" is an optional process, and is a process of retrieving a cell membrane protein in the fixed tissue. This process may be carried out after the removal process (S0101) described above. Optionally, the antigen retrieval process may be carried out simultaneously with the removal process (S0101).

The antigen retrieval is carried out by heat treatment, enzymatic treatment, or a combination thereof. The specific method of the antigen retrieval is not particularly limited, and any method known in the art may be used. For example, the methods described in Shi et al. (J Histochemistry & Cytochemistry, 2011, 59: 13-32), D'Amico et al. (J Immunological Methods, 2009, 341: 1-18), and McNicoll and Richmond (Histopathology, 1998, 32: 97-103) may be used in this process. Whether or not this process is required, and the treatment to be carried out when this process is carried out, may be appropriately selected depending on, for example, the type of the antigen and the properties of the fixed tissue.

This process comprises, for example, an enzymatic treatment step and/or a heat treatment step, and preferably comprises a heat treatment step.

<Enzymatic Treatment Step>

The enzymatic treatment may be carried out using a protease such as trypsin, DNase, proteinase K, pepsin, pronase, or ficin. The treatment conditions are not particularly limited as long as the enzyme can exhibit its activity. For example, the treatment conditions may be determined based on, for example, the optimum temperature and the optimum pH of the enzyme.

<Heat Treatment Step>

The heat treatment step may be carried out by applying a heated antigen retrieval solution to the fixed tissue. The type of antigen retrieval solution is not particularly limited, and includes, for example, citrate buffer; solutions comprising a chelating agent for divalent ions, such as EDTA; tris(hydroxymethyl)methylamine (TRIS) buffer; 4-2-hydroxyethyl-1-piperazineethanesulfonic acid (HEPES) buffer; 2-{[tris(hydroxymethyl)methyl]amino } ethanesulfonic acid (TES) buffer; 2-(*N*-morpholino)ethanesulfonic acid (TAPS) buffer; *N,N*-bis(2-hydroxyethyl)glycine (Bicine) buffer; *N*-tris(hydroxymethyl)methylglycine (Tricine) buffer; glycine-HCl buffer; periodic acid solution; urea solution; and lead thiocyanate solution. For example, an acidic compound, such as potassium dihydrogen phosphate, boric acid, diethylbarbituric acid, piperazine-*N,N'*-bis(2-ethanesulfonic acid), dimethylarsinic acid, or 2-(N-morpholino)ethanesulfonic acid, or a combination thereof, may be added to the buffers.

The pH is not particularly limited, and may be, for example, within the range of 6 to 11, 7 to 10, 7.5 to 9.5, or 8 to 9.

The temperature for the heat treatment is not particularly limited, and may be within the temperature range of, for example, 90°C to 130°C. Specifically, for example, the temperature may be within the range of 90°C to 130°C, 92°C to 128°C, 95°C to 125°C, or 99°C to 121°C. The heating time is not particularly limited. For example, the treatment may be carried out for 10 minutes or more, 20 minutes or more, 25 minutes or more, 30 minutes or more, 35 minutes or more, or 40 minutes or more. Further, for example, the time may be 3 hours or less, 2 hours or less, 90 minutes or less, 60 minutes or less, 50 minutes or less, or 45 minutes or less.

During this process, the conditions may be constant, or may change intermittently or continuously. When the conditions change, the change may be either an artificial change or a natural change. Further, the solution may be allowed to flow as appropriate. In this case, the flow may be artificially generated by an operation such as stirring, or may occur naturally due to convection or the like. Further, the fixed tissue may be moved as appropriate by, for example, changing the position of the fixed tissue.

This process may be carried out as a plurality of times separately. In this case, the conditions may be the same or different among the antigen retrievals.

1-3-3. Cooling Process

The "cooling process" is an optional process, and is a process of cooling the fixed tissue. This process may be carried out after the antigen retrieval process.

For example, when the antigen retrieval has been carried out by heat treatment, or when the temperature has been increased for the enzymatic treatment, this process may be carried out.

The cooling method is not particularly limited as long as the temperature of the fixed tissue can be lowered to a certain temperature. For example, a method in which the fixed tissue is left to stand in an atmosphere or a solution at low temperature, a method using a cooling device such as a refrigerator or a freezer, or a combination thereof may be used.

The temperature after the cooling is not particularly limited. For example, the temperature may be a freezing temperature (0°C or less), refrigeration temperature (0°C to 5°C), room temperature (1°C to 30°C), or normal temperature (15°C to 25°C). Preferably, the fixed tissue is cooled to room temperature or normal temperature.

The cooling rate and the cooling time are not particularly limited. The cooling time in this process refers to the time required for the temperature of the fixed tissue to decrease to the desired temperature. Therefore, when the temperature has been decreased to reach equilibrium and then kept constant, the time to reach the equilibrium is regarded as the duration of this process. When necessary, the fixed tissue may be stored for any length of time, for example, by leaving the fixed tissue to stand after any process including this process.

In general, the larger the difference between the temperature at the start of the cooling and the temperature of the environment in which the fixed tissue is placed, the faster the cooling rate, and the shorter the cooling time. The cooling time may be, for example, 10 minutes to 24 hours, 20 minutes to 16 hours, 30 minutes to 12 hours, 35 minutes to 6 hours, 40 minutes to 3 hours, 40 minutes to 2 hours, or 40 minutes to 1 hour.

In this process, the solution and the atmosphere may be allowed to flow as appropriate. Further, in this case, the flow may be artificially generated by an operation such as stirring, or may occur naturally due to convection or the like. Further, the fixed tissue may be moved as appropriate by, for example, changing the position of the fixed tissue.

1-3-4. Nonspecific Reaction Suppressing Process

The "nonspecific reaction suppressing process" is an optional process, and is a process of bringing the fixed tissue into contact with a reaction suppressing agent to suppress nonspecific reaction during the antibody reaction and/or the coloring (color emerging) reaction. This process may be carried out after the embedding medium removal process. When the antigen retrieval process is carried out, this process may be carried out simultaneously with or after the antigen retrieval process. When the cooling process is carried out, this process may be carried out simultaneously with or after the cooling process.

This process is broadly divided into a blocking step of suppressing nonspecific binding of an antibody, and an endogenous reaction suppressing step of suppressing nonspecific coloring reaction. Only one of these steps may be carried out, or both steps may be carried out. Each step may be independently carried out a plurality of times, and one or a plurality of other step(s) may be carried out between the steps.

<Blocking Step>

In the blocking step, the fixed tissue is brought into contact with a blocking solution to suppress the nonspecific binding of the antibody used.

The time when this step is carried out is not particularly limited as long as it is after the embedding medium removal process. When the antigen retrieval process is carried out, this step may be carried out simultaneously with or after the antigen retrieval process. For example, this step may be carried out before, simultaneously with, or after the labeling process described later. For example, this step may be carried out before, simultaneously with, or after the primary antibody reaction step and the secondary antibody reaction step. This step may be carried out a plurality of times. In this case, these steps may be carried out continuously with each other, or one or a plurality of step(s) may be included therebetween.

As the method of the blocking, any method known in the art may be used. Usually, this step is carried out by incubating the fixed tissue for a certain length of time with the fixed tissue being in contact with a blocking solution. For example, when the fixed tissue is placed on a glass slide, the blocking solution may be applied on the fixed tissue, and then incubated for a certain length of time. Further, for example, when the fixed tissue is floating in a solution, the solution may be replaced with a blocking solution, and incubated for a certain length of time.

The blocking solution generally comprises protein. The type of the protein contained in the blocking solution is not particularly limited. The protein includes, for example, animal serum protein, immunoglobulin protein, skim milk, non-fat milk, casein, and mixtures thereof.

For example, the animal from which the serum protein is derived is not particularly limited. The serum protein includes, for example, those of goats, horses, humans, mice, rabbits, rats, and pigs. Specifically, for example, bovine serum albumin, fetal bovine serum, equine serum, or goat serum may be used.

The solvent for the blocking solution is not particularly limited, and is usually a buffer. The specific type of the buffer is not particularly limited. For example, the buffer used may be a phosphate buffer (such as PBS). In particular embodiments, the buffer used may be tris(hydroxymethyl)methylamine (TRIS) buffer, 2-(*N*-morpholino)ethanesulfonic acid (TAPS) buffer, *N*,*N*-bis(2-hydroxyethyl)glycine (Bicine) buffer, *N*-tris (hydroxymethyl)methylglycine (Tricine) buffer, 4-2-hydroxyethyl-1-piperazineethanesulfonic acid (HEPES) buffer, 2-{[tris(hydroxymethyl)methyl] amino }ethanesulfonic acid (TES) buffer, acetate buffer, carbonate buffer, citrate buffer, or a combination thereof.

The solvent may comprise an additive as appropriate. For example, a surfactant such as Tween 20 or Triton X-100 may be added thereto. For example, a buffer in which these additives are preliminarily added, such as PBS-T (PBS supplemented with 0.05% Tween 20), may be used.

The pH of the solvent is not particularly limited. Specifically, the pH includes, for example, 6 to 8, 7 to 8, 7.2 to 7.8, 7.3 to 7.6, and 7.4 to 7.5.

The concentration of the protein is not particularly limited. For example, the concentration may be 0.1% to 30%, 1% to 30%, 5% to 30%, or 10% to 20% in terms of the weight/volume percent.

<Endogenous Reaction Suppressing Step>

In the endogenous reaction suppressing step, the fixed tissue is brought into contact with a reaction suppressing agent for the coloring reaction to suppress nonspecific reaction.

The time when this step is carried out is not particularly limited as long as it is after the embedding medium removal process and before the coloring step described later. For example, when the antigen retrieval process is carried out, this step may be carried out simultaneously with or after the antigen retrieval process and before the coloring step described later. For example, when the blocking step is carried out, this step may be carried out before, simultaneously with, or after the blocking step, or may be carried out before, simultaneously with, or after the labeling process described later. For example, the endogenous reaction suppressing step may be carried out before, simultaneously with, or after the primary antibody reaction step and the secondary antibody reaction step. This step may be carried out a plurality of times, and, in this case, these steps may be carried out continuously with each other, or one or a plurality of step(s) may be included therebetween.

The method for suppressing the endogenous reaction is not particularly limited as long as the method enables suppression of an endogenous reaction of the fixed tissue that potentially affects the coloring reaction to inhibit the detection of the label bound to the primary antibody described below. For example, the method may be appropriately determined according to the type of the label used and the coloring reaction used.

Specifically, for example, when a fluorescent substance is used as the label, a quenching agent may be applied to the fixed tissue in order to suppress the autofluorescence of the fixed tissue.

Further, for example, when peroxidase is used as the label, $H_2O_2$ (such as 3% $H_2O_2$), a peroxidase suppressing factor, a peroxidase suppressing reagent (such as Peroxidase Block (manufactured by DAKO)), or the like may be applied in order to suppress the peroxidase activity of the fixed tissue.

For example, when biotin is used as the label, avidin and biotin in excess amounts may be applied to the fixed tissue in order to reduce the biotin contained in the fixed tissue.

For example, when phosphatase is used as the label, a phosphatase-inhibiting agent (such as levamisole) may be applied to the fixed tissue in order to suppress the phosphatase activity of the fixed tissue.

Alternatively, this step may be carried out using a reagent(s) having 2 or more of these activities.

The length of time of this step is not particularly limited. For example, the length of time may be 15 minutes or more, 30 minutes or more, 40 minutes or more, or 60 minutes or more. Further, for example, the length of time may be 24 hours or less, 16 hours or less, 12 hours or less, 6 hours or less, 3 hours or less, or 2 hours or less.

The temperature during this step is not particularly limited. For example, the step may be carried out under a temperature condition such as freezing temperature (0°C or less), refrigeration temperature (0°C to 5°C), room temperature (1°C to 30°C), or normal temperature (15°C to 25°C).

During this step, the conditions may be constant, or may change intermittently or continuously. When the conditions change, the change may be either an artificial change or a natural change.

1-3-5. Labeling Process (S0103)

The "labeling process (S0103)" is an essential process, and is a process of binding a cell membrane protein of the fixed tissue to a labeled antibody. This process may be carried out after the embedding medium removal process (S0101). When the antigen retrieval process (S0102) is carried out, this process may be carried out simultaneously with or after the antigen retrieval process (S0102). When the nonspecific reaction suppressing process is carried out, this process may especially be carried out simultaneously with or after the blocking step.

In this process, the method for reacting the labeled antibody with the cell membrane protein in the fixed tissue is not particularly limited, and may be a method capable of directly or indirectly binding the labeled antibody to the cell membrane protein in the fixed tissue.

For example, the method may be carried out using a labeled antibody having immunological reactivity against the cell membrane protein, or may be carried out using a labeled antibody having immunological reactivity against a complex of the cell membrane protein and a binding molecule that binds to the cell membrane protein (such as an antibody having immunological reactivity against the cell membrane protein).

When a labeled antibody having immunological reactivity against the cell membrane protein is used, a step of binding the labeled antibody to the cell membrane protein is carried out for labeling. This step may be carried out after the embedding medium removal process. For example, when the antigen retrieval process is carried out, this step may be carried out simultaneously with or after the antigen retrieval process. When the nonspecific reaction suppressing process is carried out, this step may especially be carried out simultaneously with or after the blocking step.

Specific details of the antibody, the antibody solution, and the method of application of the antibody in this step are the same as those described below for the "primary antibody reaction step". Specific details of the label are the same as those described below for the "secondary antibody reaction step".

When a binding molecule that binds to the cell membrane protein is used, the following steps are typically carried out for the labeling: a first binding step of binding the binding molecule (such as an antibody having immunological reactivity to the cell membrane protein) to the membrane protein; and a second binding step of binding a labeled antibody having immunological reactivity against the complex of the binding molecule and the cell membrane protein, to the complex.

The first binding step may be carried out after the embedding medium removal process. For example, when the antigen retrieval process is carried out, the first binding step may be carried out simultaneously with or after the antigen retrieval process. When the nonspecific reaction suppressing process is carried out, the first binding step may especially be carried out simultaneously with or after the blocking step. The second binding step may usually be carried out after the first binding step. Optionally, the first binding step may be carried out simultaneously with the second binding step.

The binding steps may be carried out 3 or more times. In this case, the second and later binding steps are carried out using binding molecules capable of binding to a complex formed by an earlier binding step. In this case, the binding molecules used in the binding steps other than the last binding step (which corresponds to the second binding step) may be labeled, or may be an unlabeled binding molecule.

The type of the binding molecule is not particularly limited, and is preferably a binding molecule that specifically binds to the cell membrane protein. For example, an antibody, an aptamer, a cyclic peptide, a receptor or a ligand of the cell membrane protein, or a combination thereof may be used. The binding mode between the binding molecule and the cell membrane protein is not particularly limited. The binding molecule binds to, for example, a transmembrane domain of the cell membrane protein; a portion in the cell membrane protein other than a transmembrane domain, such as a portion not embedded in the cell membrane; or an intracellular domain or an extracellular domain of the cell membrane protein. Specific details of the binding properties of the binding molecule, the reaction solution, and the application method are the same as those described below for the "primary antibody reaction step".

The binding mode between the labeled antibody having immunological reactivity against the complex of the binding molecule and the cell membrane protein, and the complex, is not particularly limited. For example, the labeled antibody may be capable of binding only after the complex is formed, or may be capable of binding to either one of the components (the cell membrane protein or the binding molecule) of the complex. Specific details of the labeled antibody are the same as those described below for the "secondary antibody reaction step".

When a primary antibody having immunological reactivity against the cell membrane protein is used as the binding molecule, and a labeled secondary antibody having immunological reactivity against the primary antibody is used as the labeled antibody having immunological reactivity against the complex, the first binding step is referred to as the primary antibody reaction step, and the second binding step is referred to as the secondary antibody reaction step.

Details of the labeling process are described below by way of example, where the primary antibody reaction step and the secondary antibody reaction step are carried out. However, as mentioned above, the labeling process does not necessarily require these steps.

<Primary Antibody Reaction Step>

The "primary antibody reaction step" is a step of reacting a primary antibody having immunological reactivity against the cell membrane protein with the cell membrane protein of the fixed tissue. This step may be carried out after the embedding medium removal process. For example, when the antigen retrieval process is carried out, this step may be carried out simultaneously with or after the antigen retrieval process. When the nonspecific reaction suppressing process is carried out, this step may especially be carried out simultaneously with or after the blocking step.

The primary antibody used in this step is not particularly limited as long as it is an antibody having immunological reactivity against the cell membrane protein to be detected. For example, the primary antibody includes antibodies capable of binding or specifically binding to the target cell membrane protein.

Specifically, the antibody may be appropriately determined based on the type of the cell membrane protein to be detected. For example, the antibody may be an antibody capable of binding to a transmembrane domain of the cell membrane protein; an antibody capable of binding to a portion of the cell membrane protein other than a transmembrane domain, such as a portion not embedded in the cell membrane; or an antibody capable of binding to an intracellular domain or an extracellular domain of the cell membrane protein.

For example, when the cell membrane protein is CAPRIN-1 protein, an anti-CAPRIN-1 antibody or an antigen-binding fragment thereof may be used. The specific anti-CAPRIN-1 antibody is not particularly limited. For example, the anti-CAPRIN-1 antibody includes those described in WO 2010/016526, WO 2011/096517, WO 2011/096528, WO 2011/096519, WO 2011/096533, WO 2011/096534, WO 2011/096535, WO 2013/018886, WO 2013/018894, WO 2013/018892, WO 2013/018891, WO 2013/018889, WO 2013/018883, WO 2013/125636, WO 2013/125654, WO 2013/125630, WO 2013/125640, WO 2013/147169, WO 2013/147176, WO 2015/020212, WO 2018/079740, and WO 2019/189780.

The higher the binding affinity of the primary antibody used in this step to the cell membrane protein, the more easily the cell membrane protein may be detected. For example, the antibody used may have a binding constant (affinity constant) $K_a$ ($k_{on}/k_{off}$) of preferably at least $10^7$ M$^{-1}$, at least $10^8$ M$^{-1}$, at least $5\times10^8$ M$^{-1}$, at least $10^9$ M$^{-1}$, at least $5\times10^9$ M$^{-1}$, at least $10^{10}$ M$^{-1}$, at least $5\times10^{10}$ M$^{-1}$, at least $10^{11}$ M$^{-1}$, at least $5\times10^{11}$ M$^{-1}$, at least $10^{12}$ M$^{-1}$, or at least $10^{13}$ M$^{-1}$.

The type of the antibody is not particularly limited. For example, the antibody may be of any class or isotype such as IgA, IgD, IgE, IgG1, IgG2a, IgG2b, IgG3, or IgM. An antigen-binding fragment may be used as the antibody. The antigen-binding fragment includes, for example, Fab, Fv, F(ab')$_2$, and Fab'. Further, an aggregate, a polymer, or a conjugate of an immunoglobulin or an antigen-binding fragment thereof may be used when appropriate as long as the immunological reactivity against the cell membrane protein is substantially maintained.

The antibody used in this step may be either a monoclonal antibody or a polyclonal antibody.

The source of the antibody is not particularly limited. For example, the source includes antibodies, non-human animal antibodies, recombinant antibodies, humanized antibodies, and chimeric antibodies. When all or part of the antibody is derived from a non-human animal antibody, the non-human animal is not particularly limited. For example, antibodies derived from mice, hamsters, rats, guinea pigs, rabbits, ferrets, goats, monkeys, and the like may be used.

The preparation method and the purification method for the antibody used in this step are not particularly limited, and any methods known in the art may be used. For example, the preparation methods and the purification methods described in the literature exemplified for the anti-CAPRIN-1 antibody may be used.

As the method of the primary antibody reaction, any method known in the art may be used. Usually, this step is carried out by incubating the fixed tissue for a certain length of time while the fixed tissue is in contact with a primary antibody solution.

The composition of the antibody solution is not particularly limited as long as it comprises the antibody, and as long as denaturation of the antibody does not occur.

The solvent for the antibody solution is not particularly limited. For example, any of a buffer, a commercially available antibody dilution, and the like may be used. The specific type of the buffer is not particularly limited. For example, a buffer exemplified for the blocking step may be used, and an additive may be added thereto when necessary. As the additive, any substance known in the art may be used. For example, the additive includes animal serum and serum proteins (such as bovine serum albumin, fetal bovine serum, and goat serum) and surfactants (such as Tween 20 and Triton X-100). The buffer may comprise a plurality of additives. For example, the buffer may comprise fetal bovine serum, goat serum, and Tween 20. The concentration of each additive is not particularly limited. For example, the concentration may be 0.1% to 30%, 1% to 30%, 5% to 30%, or 10% to 20% in terms of the weight/volume percent.

The pH of the solvent is not particularly limited. Specifically, the pH includes, for example, 6 to 8, 7 to 8, 7.2 to 7.8, 7.3 to 7.6, and 7.4 to 7.5.

The solvent may have the same composition as that of the blocking solution, or may have a composition different therefrom.

The concentration of the antibody solution is not particularly limited as long as the cell membrane protein can be detected therewith. For example, the concentration may be appropriately set according to the amount of the antigen in the fixed tissue, the type of label bound to the secondary antibody, and the like. Specifically, for example, the concentration may be 0.1 μg/mL or more, 0.2 μg/mL or more, 0.3 μg/mL or more, 0.4 μg/mL or more, 0.5 μg/mL or more, 0.6 μg/mL or more, 0.7 μg/mL or more, or 0.8 μg/mL or more. Further, for example, the concentration may be 10 μg/mL or less, 8 μg/mL or less, 7 μg/mL or less, 5 μg/mL or less, 4 μg/mL or less, 3 μg/mL or less, 2.5 μg/mL or less, or 2 μg/mL.

For the incubation, conditions at refrigeration temperature for 12 hours to 24 hours or at normal temperature for 15 minutes to 40 minutes are usually employed. The incubation conditions are not particularly limited, and any conditions known in the art may be used.

The temperature condition for the incubation may be, for example, freezing temperature (0°C or less), refrigeration temperature (0°C to 5°C), room temperature (1°C to 30°C), or normal temperature (15°C to 25°C).

The incubation time includes, for example, 15 minutes or more, 30 minutes or more, 40 minutes or more, and 60 minutes or more. Further, for example, the incubation time may be 24 hours or less, 16 hours or less, 12 hours or less, 6 hours or less, 3 hours or less, or 2 hours or less.

The humidity condition during the incubation is not particularly limited as long as the antibody solution and the fixed tissue can remain in contact with each other during the incubation. Preferably, an environment with a humidity of 100% (for example, in a moist chamber) is employed for the incubation.

During this step, the conditions may be constant, or may change intermittently or continuously. When the conditions change, the change may be an artificial change, or may be a natural change.

<Secondary Antibody Reaction Step>

The "secondary antibody reaction step" is a step of reacting a labeled secondary antibody having immunological reactivity against the primary antibody with the fixed tissue. This step may usually be carried out after the primary antibody reaction step. Optionally, this step may be carried out simultaneously with the primary antibody reaction step.

This step may be carried out in the same manner as the primary antibody reaction step, except that the labeled secondary antibody is used instead of the primary antibody.

The composition of the solvent of the antibody solution used may be the same as or different from the composition in the primary antibody reaction step.

The type of the secondary antibody is not particularly limited as long as the secondary antibody has immunological reactivity against the primary antibody. For example, when the primary antibody has a constant region, an antibody capable of binding to the constant region of the primary antibody may be used as the secondary antibody. Although the animal from which the secondary antibody is derived is not particularly limited, the secondary antibody is preferably derived from an animal different from the animal from which the primary antibody is derived.

The type of the label is not particularly limited. For example, the label includes luminescent substances, fluorescent substances, enzymes, radioactive substances, biotin, avidin, quantum dots, enzyme substrates, enzyme cofactors, enzyme inhibiting agents, dyes, and metal ions.

The luminescent substances include, for example, acridinium esters; 3-(2'-spiroadamantane)-4-methoxy-4-(3"-phosphoryloxy)phenyl-1,2-dioxetane (AMPPD); luminol and its modified products; 4-aminophthalhydrazide; various coelenterazines; and luciferin.

The fluorescent substances include, for example, fluorescent dyes such as FITC, Texas, Cy3, Cy5, Cy7, FAM, HEX, VIC, JOE, Rox, TET, Bodipy 493, NBD, TAMRA, and Alexa-Fluor dyes; and fluorescent proteins such as GFP, EGFP, BFP, and YFP.

The enzymes include, for example, proteases, peroxidases, phosphatases (such as alkaline phosphatase), sulfatases, peptidases, glycosidases, hydrolases, oxidoreductases, lyases, transferases, isomerases, ligases, and synthetases. Preferably, a peroxidase such as horseradish peroxidase, or an alkaline phosphatase, such as calf intestine alkaline phosphatase, is used.

The radioactive substances include, for example, $^{14}C$, $^{123}I$, $^{124}I$, $^{131}I$, $^{125}I$, Tc99m, $^{32}P$, $^{35}S$, and $^{3}H$.

The label may be bound to the antibody directly or through a linker or the like, or may form a complex with the antibody through a polymer carrier or the like. The specific type of the linker or the polymer carrier is not particularly limited. For example, a linker known in the art such as a peptide linker may be used. For example, as the secondary antibody herein, a complex of an enzyme as a label and an antibody, formed through a polymer carrier may be used. Further, for example, a complex of peroxidase as a label and an antibody, formed through a polymer carrier (such as Peroxidase Labelled Polymer Conjugated (manufactured by DAKO)) may be used.

The application method is not particularly limited. For example, the same method as the method of application of the blocking solution in the blocking step may be employed.

1-3-6. Coloring Process

The "coloring process" is an optional process, and is a process of exposing the fixed tissue to a reaction agent to enable detection of the label. This process may be carried out simultaneously with or after the labeling process. For example, this process may be carried out simultaneously with or after the secondary antibody reaction step. When the endogenous reaction suppressing step is carried out, this process may be carried out thereafter.

The coloring reaction refers to a reaction that makes a label detectable.

The reaction agent is not particularly limited as long as it is an agent that causes a coloring reaction of the label used. For example, an agent comprising an antibody against the label; an agent comprising a substrate, when the label is an enzyme; an agent comprising avidin, when the label is biotin; or an agent comprising biotin, when the label is avidin; may be used.

The substrate may be appropriately determined based on the enzyme used as the label. For example, when peroxidase is used as the label, the substrate includes, for example, coloring reagents such as 3,3'-diaminobenzidine (DAB), 2,2'-azinobis(3-ethylbenzothiazoline-6-sulfonic acid) (ABTS), 3-amino-9-ethylcarbazole (AEC), 3,3',5,5'-tetramethylbenzidine (TMB), Bajoran Purple, and Vina Green. When peroxidase is used as the label, a preferred coloring reagent is DAB. Further, for example, when peroxidase is used as the label, the substrate includes, for example, coloring reagents such as 5-Bromo-4-chloro-3-indolyl phosphate (BCIP), nitro blue tetrazolium chloride (NBT), naphthol AS phosphate, fuchsine, Ferangi Blue, Vulcan Fast Red, and Warp Red.

When necessary, a substance or an agent that promotes the coloring reaction may be additionally used. For example, when DAB is used, a divalent ion, such as $Ni^{2+}$ or $Co^{2+}$, may be used, or another commercially available enzyme reaction-promoting agent may also be used. Further, for example, when naphthol AS phosphate is used, a diazonium salt for forming an azo dye by the enzyme activity of naphthol may be used in combination.

A plurality of types of reaction agents may be used in combination. For example, when biotin is used as the label, avidin and a biotinylated peroxidase may be used, and a substrate of peroxidase may be additionally used.

The reaction conditions in this process are not particularly limited. For example, the reaction conditions may be appropriately determined according to the concentration of the antibody used and the assumed amount of antigen in the fixed tissue.

The reaction time is not particularly limited. For example, the reaction time may be 5 seconds or more, 10 seconds or more, 15 seconds or more, 16 seconds or more, 19 seconds or more, or 20 seconds or more. Further, for example, the treatment time may be 60 minutes or less, 50 minutes or less, 30 minutes or less, 25 minutes or less, 20 minutes or less, 10 minutes or less, 9 minutes or less, 8 minutes or less, or 7 minutes or less.

The temperature is not particularly limited. For example, the temperature may be freezing temperature (0°C or less), refrigeration temperature (0°C to 5°C), room temperature (1°C to 30°C), or normal temperature (15°C to 25°C). Preferably, the treatment temperature is room temperature or normal temperature.

When necessary, the coloring reaction may be followed by dehydration treatment. The method of the dehydration treatment is not particularly limited. For example, ethanol may be used to allow gradual dehydration, and then an organic solvent such as xylene may be used to allow further dehydration. Specifically, for example, the fixed tissue may be immersed sequentially in aqueous ethanol solutions with concentrations of 70%, 80%, 90%, 95%, and 100%. Further, this may be followed by additional dehydration using xylene.

1-3-7. Label Detecting Process (S0104)

The "label detecting process (S0104)" is an essential process, and is a process of detecting a label bound to a cell membrane protein. This process may be carried out after the labeling process (S0103). When the coloring process is carried out, this process may be carried out simultaneously with or after the coloring process. Optionally, this process may be carried out simultaneously with the labeling process (S0103), for example, with the secondary antibody reaction step.

The detection may be preceded by any other additional staining. For example, cell nuclei may be stained. The type of the other staining is not particularly limited, and includes, for example, DAPI staining, hematoxylin-eosin (HE) staining, Gomori methenamine silver staining (GMS), periodic acid-Schiff (PAS) dye, trichrome blue staining, Masson's trichrome staining, Prussian blue staining, Giemsa staining, Gram staining, Mucicarmine staining, Verhoeff-van Gieson staining, elastica staining, carbol fuchsin staining, Golgi staining, and combinations thereof.

The detection may be preceded by a mounting operation. The mounting method and the mounting agent used are not particularly limited.

The method used for the detection is not particularly limited. The detection method may be appropriately selected in accordance with the label and the reagent used, the properties of the fixed tissue, and the like, and a device required for the detection method may be used. For example, the label may be detected by visual inspection, by using a microscope (an optical microscope such as a stereomicroscope, a confocal microscope, or a fluorescence microscope), by using a detector (such as a fluorescence-activated cell sorter (FACS), a luminometer, or an absorptiometer), or by a combination thereof. When a radioactive substance is used as the label, it may be detected, for example, by autoradiography, a scintillation counter, positron emission tomography (PET), or a combination thereof.

When the label is visualized by coloring using a coloring reagent (such as a coloring reagent reactive with peroxidase), the label is preferably detected in the bright field using an optical microscope.

When necessary, another type of staining treatment may be carried out in combination. For example, one or more organelles may be stained to identify the location of the label.

1-4. Effects

According to the detection method of the present aspect, a target cell membrane protein (such as CAPRIN-1 protein) present on the cell membrane can be detected with high sensitivity and specificity.

The method of the present aspect may be manually carried out, or may be automatically carried out using an automated staining apparatus or the like.

2. Method for Determining Treatment

2-1. Summary

A second aspect of the present invention is a method for determining the treatment. The method of the present aspect includes a disease marker detecting process and a treatment determining process as essential processes. According to the method of the present aspect, treatment for the individual from whom the fixed tissue is derived can be determined.

2-2. Method

2-2-1. Disease Marker Detecting Process

The "disease marker detecting process" is a process of detecting a disease marker in an embedded fixed tissue.

This process may be carried out in the same manner as the detection method for the cell membrane protein described in the first aspect. The method described in the first aspect is different from this process in that the cell membrane protein to be detected in this process is a disease marker.

Here, the description of the disease marker is omitted since its details are described in the embedding medium removal process in the first aspect.

The individual from which the fixed tissue in the present aspect is derived is not particularly limited. For example, the individual may be any of an individual having a disease with which the disease marker to be detected is associated, an individual potentially having the disease, or a healthy individual.

2-2-2. Treatment Determining Process

The "treatment determining process" is a process of determining a treatment for the individual based on the disease marker detected by the disease marker detecting process. This process may be carried out simultaneously with or after the disease marker detecting process.

The treatment determining method is not particularly limited, and may be carried out based on the corresponding relationship between the disease marker and the treatment. For example, whether or not a disease marker has been detected in the fixed tissue may be determined, and then the treatment may be determined from the obtained determination result based on the corresponding relationship between the disease marker and the treatment.

\<Determination of Whether or Not Disease Marker Has Been Detected\>

The method for determining whether or not the disease marker has been detected is not particularly limited. For example, the disease marker may be determined to be detected when a label is detected, or when the detected label satisfies a certain condition.

Here, the condition is not particularly limited. For example, the amount of the label in fixed tissue may be quantified, and the fact that the quantified amount is a certain level or more may be used as the condition.

The quantification method is not particularly limited. For example, a score value reflecting the stained state may be estimated for digitization. The score value is classified, for example, on a 2- or higher-point scale, preferably on a 4-point scale. When the score value reflecting the stained state of the cell membrane protein expressed on the cell membrane (for example, on the cell surface) is classified on a 4-point scale, scores are set, for example, as follows.

- Score 0: The positive cell rate (the proportion of the cells for which the label is detected on the cell membrane) is less than 10%.
- Score 1: The positive cell rate is 10% or more. However, the label is limited to part of the cell membrane of diseased cells (such as cancer cells), and shows low staining intensity.
- Score 2: The positive cell rate is 10% or more. The label is localized to the cell membrane of diseased cells (such as cancer cells), and shows moderate staining intensity.
- Score 3: The positive cell rate is 10% or more. The label is localized to the cell membrane of diseased cells (such as cancer cells) and shows high staining intensity.

Such setting of the score value has been specified for cancer markers by the American Society of Clinical Oncology in the United States, and has been approved by the Japanese Society of Pathology in Japan. Further, this setting has been applied to "Hercep Test", which quantifies the abundance of a cancer antigen, Her2, present in a patient sample. Quantification of Her2 has been specified in the ASCO/CAP Her2 testing guidelines, and a Her2 testing guide including this score setting has also been specified in Japan by the Trastuzumab Pathology Committee. For example, when a protein whose abundance on the cell membrane increases on the basis of a disease is to be detected as the disease marker, the score value may be set according to this example. Further, for example, when the score is 1 or more, 2 or more, or 3 or more, the disease marker may be, but does not necessarily need to be, determined to have been detected. Preferably, the disease marker is determined to have been detected when the score is 2 or more (score 2 and 3).

The proportion of the diseased cells whose label has been detected, described for each score value, may be estimated by counting at least 500 cells within the field of view using an optical microscope while increasing its sensitivity from ×4, ×10, to ×20, measuring the cells exhibiting the staining image on the cell membrane described for each score value, and then using the following formula.

$$\text{Number of positive cells/total number of cells (about 500)} \times 100 \ (\%)$$

Further, for example, the disease marker may be determined to be detected based on a comparison between the amount of the label detected in the fixed tissue that is the detection target, and the amount of the label in a reference tissue (such as a fixed tissue without the disease or a fixed tissue with the disease). For example, when the marker is a disease marker whose expression increases based on the disease, the disease marker may be determined to be detected when the amount of the label detected is greater than, or statistically significantly greater than, the amount of the label in a fixed tissue without the disease. Conversely, in this case, for example, the disease marker may be determined not to be detected when the amount of the label detected is smaller than, or statistically significantly smaller than, the amount of the label in fixed tissue with the disease.

The term "statistically significant" herein refers to the presence of a significant difference upon statistical analysis of a quantitative difference between the two. Specifically, a significant difference is present when, for example, the critical rate (significance level) is less than 5%, less than 1%, or less than 0.1%. The test method is not particularly limited as long as it is a known method capable of determining the presence or absence of significance. For example, Student's t-test or a multiple comparison test method may be used.

Further, the detection of the disease marker may be determined based on, for example, a cut-off value for determining the detection of the disease marker. For example, when the marker is a disease marker whose expression increases based on the disease, the disease marker may be determined to be detected when the amount of the label detected exceeds the cut-off value.

<Determination of Treatment>

The method for determining the treatment is not particularly limited. For example, the treatment may be determined by comparing information on the detected disease marker with information on the correspondence relationship between the detection of various disease markers and treatment methods. In this case, information on a plurality of disease markers may be used. For example, information of disease markers detected independently from the disease marker detecting process in the present aspect (such as disease markers other than cell membrane proteins, and disease markers obtained from sources other than the fixed tissue) may be additionally used to determine the treatment.

As the information of the correspondence relationship between the detection of disease markers and treatment methods, any information known in the art may be used.

In this process, an electronic computing device such as a computer may be used to carry out one or both of the determination of the detection of the disease marker and the determination of the treatment.

2-3. Effects

According to the method of the present aspect, treatment for the individual from whom the fixed tissue is derived can be accurately determined based on whether or not the disease marker is detected from the fixed tissue.

3. Apparatus for Detecting Cell Membrane Protein

3-1. Summary

A third aspect of the present invention is an apparatus for detecting a cell membrane protein (0200). The apparatus of the present aspect comprises: an embedding medium removal unit (0210), a labeling reaction unit (0230), and a label detecting unit (0240) as essential components; and an antigen retrieval unit (0220), a cooling unit, a nonspecific reaction suppressing unit, a coloring reaction unit, and a control unit (0250) as optional components. According to the method of the present aspect, a cell membrane protein can be detected with high specificity.

3-2. Configuration

A functional block diagram of a configuration example of the production apparatus of the present invention is shown in Figure 16. The embedding medium, the fixed tissue, the disease, the cell membrane protein, and the like are as described in the first aspect. Each unit constituting the detection apparatus of the present aspect is described below.

The cell membrane protein in the apparatus of the present aspect may be, for example, a cell membrane protein expressed on the surface of a cancer cell as described in the first aspect, and may preferably be a disease marker such as a cancer marker, which may specifically be CAPRIN-1 protein or the like.

3-2-1. Embedding Medium Removal Unit (0210)

The "embedding medium removal unit (0210)" is an essential component in the apparatus of the present aspect, and is configured such that a fixed tissue comprising an embedding medium in a solid state can be brought into contact with a removal solution to remove the embedding medium. The embedding medium removal unit performs the embedding medium removal process (S0101) in the method for detecting the cell membrane protein according to the first aspect. The embedding medium removal unit comprises, for example, storage means for the removal solution (0211), contact means for the fixed tissue and the removal solution (0212), and holding means for the fixed tissue (0213).

<Storage Means (0211)>

The storage means (0211) is a container configured such that the removal solution can be stored therein. The specific configuration of the storage means for the removal solution is not particularly limited.

The overall shape of the storage means is not particularly limited as long as the shape allows the storage of a solution therein. For example, the shape may be any of a polygonal columnar shape (including a regular polygonal columnar shape and a near-polygonal columnar shape), an elliptical columnar shape (including a cylindrical liquid shape and a near-elliptical columnar shape), and a spherical shape (including a near-spherical shape).

The capacity of the container is not particularly limited, and may be appropriately set in accordance with the volume of the solution used in each time of operation by the apparatus of the present aspect. For example, when the apparatus of the present aspect is used on a laboratory scale, a capacity of about 10 mL to 200 mL may be sufficient. On the other hand, when the apparatus is used on an industrial scale, or when the apparatus is configured such that it can withstand repeated operations, the capacity may be, for example, several liters or more or several ten liters or more.

The material constituting the container is not particularly limited. However, from the viewpoint of storing a solution in the container, the material is preferably a material which has a strength that allows storage of the solution, which has a rigidity enough to maintain the shape during the storage, which does not allow penetration of the solution to the outside, and at least whose inner wall is not decomposed or degraded by the solution. For example, the material includes, but is not limited to, metals, synthetic resins (plastics), glasses, and porcelains.

Depending on the configuration of the contact means, when the fixed tissue is brought into contact with the solution in the storage means, the shape and the size of the container are not particularly limited as long as at least the portion of the fixed tissue to be subjected to the reaction can be stored in the container.

In some cases, the container may additionally be provided with an opening through which the fixed tissue is to be passed, as well as with a flow mechanism such as an opening through which a liquid is to be passed (such as an outlet and/or an inlet), a channel, a flow valve, or a stirring blade; a filtration mechanism for removing solids derived from the fixed tissue; and/or the like.

The configuration of the storage means used in the embedding medium removal unit may be the same as that of storage means used in one or more other units, or may be different from those of any storage means used in the other units.

<Contact Means (0212)>

The contact means (0212) is configured such that the fixed tissue held by the holding means (0213) can be brought into contact with the removal solution stored in the storage means (0211). The specific configuration of the contact means is not particularly limited. For example, the contact means may be means capable of spraying, sprinkling, or applying the removal solution to the fixed tissue, or may be means capable of immersing the fixed tissue in the removal solution.

When the storage means and the holding means are distantly located, the contact means may comprise an optional mechanism that reduces the distance. Such a mechanism includes, for example, a mechanism for moving the holding means and/or the storage means, a channel through which the removal solution can be transferred from the storage means to the vicinity of the holding means, and a combination thereof.

The configuration of the contact means used in the embedding medium removal unit may be the same as those of contact means used in one or more other units, or may be different from those of any contact means used in the other units. Further, the contact means may be configured such that it can be commonly used by 2 or more units.

<Holding Means (0213)>

The holding means (0213) is configured such that a fixed tissue comprising an embedding medium in a solid state can be held. The specific configuration of the holding means for the fixed tissue is not particularly limited. For example, the holding means commonly used for immunochemical staining of a fixed tissue may be used. For example, the holding means may be configured such that the fixed tissue can be gripped, pinched, or mounted, or may be configured such that the fixed tissue can be stored therein.

The configuration that allows the gripping is not particularly limited, and includes any of: a mutually integrated configuration; and a removable configuration, for example, holding means based on the magnetic force of a permanent magnet, a temporary magnet, or the like, latching means such as a hook or a string, fixation means such as a chuck, or a combination thereof.

The configuration that allows the pinching is not particularly limited, and includes both a structure for sandwiching the whole fixed tissue (such as a structure in which the fixed tissue is placed in a space sandwiched between partitions) and a structure for sandwiching part of the fixed tissue (such as a structure in which the fixed tissue is placed in an upright position between low partitions).

The configuration that allows the mounting is not particularly limited, and includes any configuration capable of placement of the fixed tissue thereon. The surface shape of the mounting surface is not particularly limited, and may be configured such that the position of the fixed tissue can be restricted, or such that the position of the fixed tissue is not restricted. Further, the surface shape may be a shape in which one or more pores are formed on the surface (such as a shape composed of linear structures that are crossing or not crossing with each other) or may be a shape without a pore. When a pore(s) is/are formed, the size, the number, and shape of the pore(s) are not particularly limited as long as the fixed tissue does not drop off.

The configuration that allows storage of the fixed tissue therein is not particularly limited, and includes any configuration in which the fixed tissue can be stored, and in which the internal space communicates with the external space. The configuration in which the internal space communicates with the external space includes both a configuration in which the communication with the external space is constantly maintained, and a configuration in which the communication with the external space occurs only under specific conditions. The configuration in which the communication with the external space is constantly maintained includes, for example, a configuration in which the fixed tissue is surrounded by, or stored in, a material in which pores are formed such as a mesh-shaped material, a fabric-shaped material, a filter-shaped material, or a combination thereof. The configuration in which the communication with the external space occurs only under specific conditions includes, for example, a configuration (such as a container) in which the fixed tissue is stored in a space formed by a material without a hole that allows entrance of an agent. In this case, for example, an opening that can be opened or closed depending on conditions may be additionally formed.

The material of the holding means is not particularly limited. For example, a fiber material, an organic resin, a glass-based material, a metallic material, or a combination thereof may be used. Further, the holding means may be composed of a rigid body, or at least part of the holding means may be composed of a soft body.

When the holding means has a portion in contact with an agent, this portion is preferably a material that is not decomposed or degraded by the agent.

Figure 16 shows an example of an embodiment in which the fixed tissue is held by one holding means until the fixed tissue reaches the label detecting unit (0240). However, each unit may comprise holding means independently of the other units regardless of whether the configuration of the holding means is the same or different. Holding means in different units may be configured such that the fixed tissue can be transferred therebetween. The configuration of the holding means used in the embedding medium removal unit may be the same as those of holding means used in one or more other units, or may be different from those of any holding means used in the other units.

<Temperature Regulating Means>

The temperature regulating means is configured such that the temperature of the fixed tissue can be controlled. In particular, the temperature regulating means herein is configured such that the fixed tissue can be maintained below the melting point of the embedding medium. The specific configuration of the temperature regulating means is not particularly limited.

For example, the temperature regulating means may be configured to enable direct control of the temperature of the fixed tissue itself, or may be configured to enable control of the temperature of the solution in the vicinity of the fixed tissue. The temperature regulating means may be configured such that the temperature can be maintained, or such that the temperature can be changed. The configuration that allows the maintenance of the temperature includes, for example, configurations utilizing an insulation material, a gas layer, a vacuum layer, or a combination thereof. When the temperature regulating means is configured such that the temperature can be changed, it may be configured as a single means capable of both heating and cooling, or capable of only one of these. Specifically, for example, the temperature regulating means includes thermostats, heaters and coolers for liquids or solids, exothermic agents and endothermic agents utilizing chemical reactions, and storage tanks for hot or cold solutions. One or more of the holding means (0213), the contact means (0212), and the storage means (0211) may be equipped with the temperature regulating means, or the temperature regulating means may be included separately from these.

Preferably, by the temperature regulating means, the fixed tissue is maintained below the melting point of the embedding medium. The temperature to be achieved by the regulation is not particularly limited, and may be appropriately set in accordance with the type of embedding medium. The temperature is the same as described in the first aspect.

<Washing Means>

Washing means is configured such that the surface of the fixed tissue can be washed. The specific configuration of the washing means is not particularly limited. For example, the washing means may be configured such that the washing solution described in the first aspect can be stored or supplied. For example, the configuration that allows the storage is the same as that of the storage means (0211) mentioned above, except that the liquid to be stored is the washing solution rather than the removal solution.

The configuration of the washing means used in the embedding medium removal unit may be the same as those of the washing means used in one or more other units, or may be different from those of any washing means used in the other units. Further, the washing means may be configured such that it can be commonly used by 2 or more units.

3-2-2. Antigen Retrieval Unit (0220)

The antigen retrieval unit (0220) is an optional component in the apparatus of the present aspect, and is configured such that the cell membrane protein in the fixed tissue that has been subjected to the embedding medium removal unit (0210) can be retrieved. The antigen retrieval unit (0220) performs the antigen retrieval process (S0102) in the method for detecting the cell membrane protein according to the first aspect. The antigen retrieval unit (0220) usually comprises antigen retrieval means (0221), contact means (0222), holding means for the fixed tissue, and washing means.

The contact means (0222), the holding means, and the washing means are the same as those described for the embedding medium removal unit (0210).

<Antigen Retrieval Means (0221)>

The antigen retrieval means (0221) is configured such that it retrieves the cell membrane protein in the fixed tissue. The specific configuration of the antigen retrieval means (0221) is not particularly limited, and may be appropriately selected in accordance with the type of the antigen retrieval treatment to be performed.

For example, when the antigen retrieval treatment is based on an enzymatic treatment step, storage means for the enzyme liquid described in the first aspect may be employed, and, when the antigen retrieval treatment is based on a heat treatment step, storage means for the antigen retrieval solution described in the first aspect may be employed. The storage means for the liquids are the same as the storage means for the removal solution (0211), except that the solution to be stored is the enzyme liquid or the antigen retrieval solution.

The antigen retrieval means is preferably configured such that the temperature of the liquid can be controlled. Its specific configuration is not particularly limited. The configuration is the same as that of the temperature regulating means in the embedding medium removal unit (0210), except that the temperature to be achieved by the regulation is the temperature in the antigen retrieval process described in the first aspect. Even when only one of the processes is carried out in one time of operation, storage means for the solutions corresponding to both processes may be provided. Further, storage means for a plurality of types of solutions may be provided for each of the enzyme liquid and the antigen retrieval solution.

3-2-3. Cooling Unit

The cooling unit is an optional component in the apparatus of the present aspect, and is configured such that the fixed tissue that has been subjected to the antigen retrieval unit (0220) can be cooled. The cooling unit performs the cooling process in the method for detecting the cell membrane protein according to the first aspect. The cooling unit usually comprises cooling means and holding means for the fixed tissue, and also comprises, in some cases, contact means and washing means.

The holding means, the contact means, and the washing means are the same as those described for the embedding medium removal unit (0210).

<Cooling Means>

The cooling means is configured such that the temperature of the fixed tissue that has been brought into contact therewith can be decreased. The specific configuration is not particularly limited as long as the temperature of the fixed tissue can be decreased. For example, the cooling means may be configured such that heat can be radiated from the fixed tissue, or such that heat can be absorbed from the fixed tissue. There is no particular limitation of the specific configuration, and the configuration is basically the same as that of the temperature regulating means in the embedding medium removal unit (0210), except that the configuration is for the purpose of cooling, and that the temperature to be achieved by the regulation is the temperature in the cooling process in the first aspect. Means also capable of heating may be employed to use only the cooling function thereof.

In particular, in the cooling herein, the fixed tissue may be brought into contact with any solid, solution, or gas, or a combination thereof. Thus, the cooling means may be configured such that the fixed tissue can be brought into contact with a solid, solution, gas, or the like at a temperature lower than that of the fixed tissue.

3-2-4. Nonspecific Reaction Suppressing Unit

The nonspecific reaction suppressing unit is an optional component in the apparatus of the present aspect, and is configured such that the fixed tissue that has been subjected to the embedding medium removal unit (0210) can be brought into contact with a reaction suppressing agent. In some cases, the nonspecific reaction suppressing unit may be configured such that the fixed tissue that has been subjected to the antigen retrieval unit (0220) and/or the cooling unit can be treated. The nonspecific reaction suppressing unit performs the nonspecific reaction suppressing process in the method for detecting the cell membrane protein according to the first aspect. The nonspecific reaction suppressing unit usually comprises reaction suppressing agent storage means, contact means, and holding means for the fixed tissue, and also comprises, in some cases, washing means.

The contact means, the holding means, and the washing means are the same as those described for the embedding medium removal unit (0210).

The specific configuration of the reaction suppressing agent storage means is not particularly limited, and may be appropriately selected in accordance with the type of the nonspecific reaction suppressing treatment to be performed. For example, when the nonspecific reaction suppressing treatment is based on the blocking step, storage means for the blocking solution described in the first aspect may be employed, and, when the nonspecific reaction suppressing treatment is based on the endogenous reaction suppressing step, storage means for the reaction suppressing agent for the coloring reaction described in the first aspect may be employed. The storage means for the liquids are the same as the storage means for the removal solution except that the solution to be stored is the reaction suppressing agent. Even when only one of the steps is carried out in one time of operation, storage means for the solutions corresponding to both steps may be provided. Further, storage means for a plurality of types of reaction suppressing agents may be provided.

3-2-5. Labeling Reaction Unit (0230)

The labeling reaction unit (0230) is an essential component in the apparatus of the present aspect, and is configured such that the cell membrane protein in the fixed tissue that has been subjected to the embedding medium removal unit (0210) can be reacted with a labeled antibody having immunological reactivity against the cell membrane protein. In some cases, the labeling reaction unit may be configured such that the fixed tissue that has been subjected to the antigen retrieval unit (0220), the cooling unit, and/or the nonspecific reaction suppressing unit can be treated. The labeling reaction unit (0230) performs the labeling process (S0103) in the method for detecting the cell membrane protein according to the first aspect. The labeling reaction unit (0230) usually comprises labeled-antibody storage means (0233), contact means therefor (0234), and holding means for the fixed tissue, and also comprises, in some cases, binding-molecule storage means (0231), contact means therefor (0232), and washing means.

The contact means (0232, 0234), the holding means, and the washing means are the same as those described for the embedding medium removal unit (0210).

When a plurality of times of binding steps are carried out, the labeling reaction unit may comprise binding-molecule storage means (0231) for storing the binding molecule used in each binding step. Specifically, for example, when the primary antibody reaction step and the secondary antibody reaction step are carried out, the unit may comprise storage means for the primary antibody solution described in the first aspect as the binding-molecule storage means (0231), and storage means for the secondary antibody solution described in the first aspect as the labeled-antibody storage means (0233).

The storage means for each solution is the same as the storage means for the removal solution (0211) except that the solution to be stored is the antibody solution or the solution of the binding molecule described in the first aspect. Even when only the binding reaction by the labeled antibody is carried out in one time of operation, storage means for the solution of the binding molecule may be provided. Further, storage means for a plurality of types of antibodies or binding molecules may be provided.

3-2-6. Coloring Reaction Unit

The coloring reaction unit is an optional component in the apparatus of the present aspect, and is configured such that the fixed tissue that has been subjected to the labeling reaction unit (0230) can be exposed to the reaction agent to thereby enable detection of the label. The coloring reaction unit performs the coloring process in the method for detecting the cell membrane protein according to the first aspect. The coloring reaction unit usually comprises a reaction agent storage unit, contact means, and holding means for the fixed tissue, and also comprises, in some cases, washing means.

The contact means, the holding means, and the washing means are the same as those described for the embedding medium removal unit (0210). The storage means for the reaction agent is the same as the storage means for the removal solution (0211), except that the solution to be stored is the reaction agent described in the first aspect.

3-2-7. Label Detecting Unit (0240)

The label detecting unit (0240) is an essential component in the apparatus of the present aspect, and is configured such that the labeled antibody in the labeled-antibody-bound fixed tissue that has been subjected to the labeling reaction unit can be detected. The label detecting unit (0240) performs the label detecting process (S0104) in the method for detecting the cell membrane protein according to the first aspect. The label detecting unit usually comprises detection means (0241), analysis means (0242), and holding means for the fixed tissue.

The holding means is the same as the holding means (0213) in the embedding medium removal unit (0210).

<Detection Means (0241)>

The detection means (0241) is configured such that the label bound to the cell membrane protein in the fixed tissue can be detected. The configuration of the detection means (0241) is not particularly limited, and may be appropriately selected in accordance with the label and the reagent used, the properties of the fixed tissue, and the like.

Specifically, for example, the detection means may comprise a microscope (an optical microscope such as a stereomicroscope, a confocal microscope, or a fluorescence microscope), a detector (such as a fluorescence-activated cell sorter (FACS), a luminometer, or an absorptiometer), autoradiography, a scintillation counter, positron emission tomography (PET), or a combination thereof. When necessary, the detection means may comprise a plurality of types of detection means.

<Analysis Means (0242)>

The analysis means (0242) is configured such that signal data of the label detected by the detection means (0241) can be analyzed. The configuration of the analysis means (0242) is not particularly limited, and may be appropriately selected in accordance with the detection means (0241) used.

For example, analysis means that is usually used for analysis of signal data using each detection means may be provided, or an analysis mechanism included in the detection means used may be used as the analysis means in the present aspect. Typically, the analysis means includes a computer. When the analysis means includes a computer, there is no limitation of the specific configurations of the hardware means and the software means. For example, the later-described configuration may be employed for the control unit.

Specific details of the analysis process performed herein are not particularly limited. For example, the process of converting the signals to image data or the process of removing noise from the signal data may be carried out. When necessary, the analysis means may be configured to enable the process of determining whether or not the disease marker described in the second aspect has been detected.

The analysis means may also comprise an output mechanism that displays an analysis result to a user.

3-2-8. Control Unit (0250)

The control unit (0250) is an optional component in the apparatus of the present aspect, and is configured such that the communication between each unit and the fixed tissue, and the treatment conditions in each unit, can be controlled. For example, the control unit (0250) allows the fixed tissue to communicate with a predetermined unit at a predetermined time such that the fixed tissue is treated under predetermined treatment conditions (regarding the type of the reaction solution, the temperature conditions, and the like) for a predetermined length of time.

The control unit may be configured such that the series of methods described in the first aspect can be carried out by the apparatus of the present aspect, or such that part of the processes of the methods described in the first aspect can be carried out. Typically, the control unit comprises a computer.

The control unit is composed of hardware means, or both hardware means and software means. The hardware means is composed of, for example, a CPU (0251), a volatile memory (0252), a nonvolatile memory (0253), an interface (0254), a system bus connecting these (0255), and a peripheral apparatus, which are constituents thereof. The peripheral apparatus is not particularly limited, and includes, for example, a timepiece and a water volume sensor. The software means is composed of, for example, a program executable on a hardware memory.

In the control unit, for example, various programs (such as a communication control program and a treatment condition control program) stored in the nonvolatile memory (0253) of the hardware means are loaded into the volatile memory (0252), and the programs are sequentially executed. By this, data in the memories and data input via the interface (0254) are, for example, processed, stored, and output to realize the function of each unit. For example, when a programmed predetermined time is reached, the contact means may be activated to bring the fixed tissue into contact with a desired agent or the like, and, after a certain time has passed, the contact means may be activated again to stop the contact. Further, for example, when a programmed predetermined time is reached, the temperature regulating means or the cooling means may be activated to adjust the temperature of the fixed tissue to a predetermined temperature, and, after a certain time has passed, the contact means may be activated again to stop the contact. Furthermore, when, during the reaction in each unit, information indicating deviation from desired conditions is input from a sensor such as a temperature sensor or a pH sensor via the interface (0254), a reaction condition control program loaded in the volatile memory (0252) may be executed to add an agent for adjusting the conditions within a desired range, or to activate the temperature regulating means.

When necessary, a display unit that presents information to a user may be provided, and a display control unit controls the content to be presented. The hardware of the display unit is not particularly limited, and includes, for example, a display.

Further, when necessary, an input device with which a user inputs information may be provided. The input device includes, for example, a keyboard, a mouse, a touch panel, and a touch pen. The information input from the input device is controlled by an input/output control unit. The input/output control unit controls input of data and instructions from the input device, as well as output of various data from a processing apparatus. The information that the user is requested to input is not particularly limited. For example, the user may be requested to set the length of time and the reaction conditions of each process, to specify which process(es) is/are carried out, and to specify criteria (such as the turbidity and the duration) for renewal of the solution in the storage means.

Examples

The present invention is described below more specifically based on Examples. However, the scope of the present invention is not particularly limited by these Examples.

Example 1: Detection of CAPRIN-1 Protein by Immunohistochemical Staining (1)

(Paraffin Removal Process)

Paraffin was removed from various paraffin-embedded human cancer tissue sections with a thickness of 4 $\mu$m (breast cancer tissues, lung cancer tissues, pancreatic cancer tissues, renal cancer tissues, colon cancer tissues, gastric cancer tissues, ovarian cancer tissues, and prostate cancer tissues; manufactured by US BIOMAX Inc.; sections derived from different specimens of 1361 patients were used for each) using xylene for 20 minutes. The xylene was replaced 3 times at 5-minute intervals. Subsequently, 100%, 90%, and 80% aqueous ethanol solutions were used instead of xylene to perform washing for 5 minutes each by the same procedure. These operations were carried out at room temperature. Thereafter, PBS-T (phosphate buffer solution (PBS) supplemented with 0.05% Tween 20; pH 7.4) was used to perform 3 times of washing for 5 minutes each.

(Antigen retrieval Process)

The various human cancer tissue sections that had been subjected to the paraffin removal step were placed in staining jars filled with 10 mM citrate buffer (pH 6.0) supplemented with 0.05% Tween 20, and subjected to heat treatment at 99°C for 40 minutes. Thereafter, the sections were allowed to cool by being left to stand at room temperature for 40 minutes or more.

(Nonspecific Reaction Suppressing Process)

(Endogenous Reaction Suppressing Step for Peroxidase)

Excessive water was removed from the vicinity of the sections of various human cancer tissues that had been subjected to the antigen retrieval process, and then an appropriate amount of Peroxidase Block (manufactured by DAKO) was added dropwise to the sections, followed by leaving the sections to stand at room temperature for 5 minutes.

(Blocking of Protein)

The sections were washed 3 times with PBS-T for 5 minutes each, and then PBS-T solution supplemented with 10% FBS was added thereto as a blocking liquid, followed by incubating the sections at room temperature for 1 hour in a moist chamber.

(Labeling Process: Primary Antibody Reaction Step)

A primary antibody solution comprising a mouse anti-CAPRIN-1 monoclonal antibody prepared in WO 2013/018891 was added to the sections of various human cancer tissues that had been subjected to the nonspecific reaction suppressing process, and then the sections were incubated at 4°C overnight in a moist chamber. In the preparation of the primary antibody solution, the mouse anti-CAPRIN-1 antibody mentioned above was added to PBS-T solution supplemented with 5% FBS and 20% normal goat serum, to a final concentration of 2 $\mu$g/mL.

(Labeling Process: Secondary Antibody Reaction Step)

The sections of various human cancer tissues that had been subjected to the primary antibody reaction step were washed 3 times with PBS-T for 10 minutes, and an appropriate amount of Peroxidase Labelled Polymer Conjugated (manufactured by BIOCARE) was added dropwise to the sections, followed by incubating the sections at room temperature for 30 minutes in a moist chamber.

(Coloring Process)

The sections of various human cancer tissues that had been subjected to the secondary antibody reaction step were washed 3 times with PBS-T for 10 minutes, and DAB coloring solution (manufactured by BIOCARE) was added to the sections, followed by leaving the sections to stand at room temperature for about 20 seconds to allow coloring. Thereafter, the coloring solution was discarded, and then the sections were washed 3 times with PBS-T for 10 minutes, followed by rinsing the sections with distilled water. Thereafter, the sections were immersed in aqueous ethanol solutions at concentrations of 70%, 80%, 90%, 95%, and 100% sequentially for 1 minute each, and then incubated in xylene overnight to perform dehydration treatment.

(Mounting)

The sections of various human cancer tissues that had been subjected to the coloring step were mounted using cover glasses and Glycergel Mounting Medium (manufactured by BIOCARE) as a mounting agent.

(Observation)

Staining images of CAPRIN-1 protein in cancer cells in the cancer tissues of the mounted sections were observed using a $4\times$ objective lens for an optical microscope.

Staining images obtained from ovarian cancer tissue are shown in Figure 1. In the figure, panel A shows a staining image of cell nuclei and CAPRIN-1 protein, and panel B shows a staining image of CAPRIN-1 protein after exclusion of the signals of the cell nuclei. As indicated by the arrows in the figure, signals of CAPRIN-1 protein localized on the cell membrane were found along the borders of the cells (Figure 1B).

It was thus found that this staining method enables highly accurate detection of signals of the cell membrane protein on the cell membrane.

Further, it was found that, when the mouse anti-CAPRIN-1 monoclonal antibody or the rabbit anti-CAPRIN-1 monoclonal antibody prepared in WO 2010/016526, WO 2011/096517, WO 2011/096528, WO 2011/096519, WO 2011/096533, WO 2011/096534, WO 2013/018894, WO 2013/018892, WO 2013/018891, WO 2013/018889, WO 2013/018883, WO 2013/125636, WO 2013/125654, WO 2013/125630, WO 2013/125640, or WO 2015/020212 is applied as the antibody used in the primary antibody reaction step in the present Example, signals of the cell membrane protein on the cell membrane can be accurately detected to the same level as in the above-described case.

Comparative Example 1: Detection of CAPRIN-1 Protein by Immunohistochemical Staining

Paraffin-embedded sections of various human cancer tissues (manufactured by BIOMAX Inc.) derived from the same specimens as in Example 1 were subjected to immunohistochemical staining in the same manner as in the processes described in Example 1, except that the removal of paraffin in the paraffin removal process was preceded by heat treatment (baking) at 62°C for 15 minutes, and that the heat treatment in the antigen retrieval process was carried out at 121°C.

Staining images obtained from ovarian cancer tissue are shown in Figure 2. As indicated by the arrowheads in the figure, signals of CAPRIN-1 protein were found not only in the cell membrane, but also in the cytoplasm despite the fact that CAPRIN-1 protein is localized on the cell membrane. Thus, the cells appeared as if they were painted over by the signals (Figure 2B).

It was therefore found that a remarkable decrease in the detection accuracy of the signals of the cell membrane protein on the cell membrane occurs when, unlike the staining method of Example 1, the baking is carried out.

Example 2: Determination of CAPRIN-1-Positive Cancer Cells

First, staining images of CAPRIN-1 protein, and the signal intensities of positive stains, in cancer cells in the cancer tissues of the sections mounted in Example 1 and Comparative Example 1 were observed using a $\times4$ objective lens for an optical microscope. Subsequently, the objective lens was switched to a $10\times$ or $20\times$ lens, and the proportion of cancer cells showing color development specifically in the cell membrane among all cells of the cancer tissue in all sections derived from each specimen (CAPRIN-1-positive cell rate) was calculated.

Based on the CAPRIN-1-positive cell rate and localization of CAPRIN-1 signals, each cancer tissue specimen was scored on a scale of 0 to 3 as follows. According to the scoring, the specimen was determined to be a CAPRIN-1-positive cancer tissue specimen when the score was 2 or 3.

- Score 0 (no overexpression of CAPRIN-1 protein): The CAPRIN-1-positive cell rate (the proportion of cells showing cell-membrane-specific signals) is less than 10%.
- Score 1 (no overexpression of CAPRIN-1 protein): CAPRIN-1-positive cell rate is 10% or more, but the cells show signals limited to part of the cell membrane, and show low staining intensity.
- Score 2 (overexpression of CAPRIN-1 protein): CAPRIN-1-positive cell rate is 10% or more, and the cells show signals localized to the cancer cell membrane, and show moderate staining intensity.
- Score 3 (overexpression of CAPRIN-1 protein): The CAPRIN-1-positive cell rate is 10% or more, and the cells show signals localized to the cancer cell membrane, and show high staining intensity.
- Tissue specimens with a score of 2 or 3 are determined to be CAPRIN-1-positive cancer tissue specimens.

Figures 3 to 10 show exemplary staining images for each score. For example, for gastric cancer tissues, as shown in Figure 2, specimens with a score 0 (Figure 3) and specimens with a score 1 (Figure 4) were determined to be CAPRIN-1-negative specimens. In these specimens, the signals on the cell membrane were weak, and the boundaries between the cells were unclear. On the other hand, for gastric cancer tissues, as shown in Figures 5 and 6, specimens with a score 2 (Figure 5) and specimens with a score 3 (Figure 6) were determined to be CAPRIN-1-positive specimens. These specimens showed strong signals on the cell membrane, and the boundaries between the cells were clear.

Other cancer tissues were also subjected to the determination in the same manner. For example, for renal cancer tissues, as shown in Figures 7 and 8, specimens with a score 0 (Figure 7) and specimens with a score 1 (Figure 8) showed weak signals on the cell membrane, and hence were determined to be CAPRIN-1-negative specimens. On the other hand, as shown in Figures 9 and 10, specimens with a score 2 (Figure 9) and specimens with a score 3 (Figure 10) showed strong signals on the cell membrane, and hence were determined to be CAPRIN-1-positive specimens.

According to the determination results, the proportion of CAPRIN-1-positive cancer tissue specimens among the specimens stained in Example 1, that is, the proportion of CAPRIN-1-positive cancer tissue specimens among the specimens of each type of cancer tissue, was 75% for breast cancer tissues, 65% for lung cancer tissues, 70% for pancreatic cancer tissues, 70% for renal cancer tissues, 60% for colon cancer tissues, 60% for gastric cancer tissues, 65% for ovarian cancer tissues, and 90% for prostate cancer tissues.

On the other hand, according to the determination results based on the specimens stained in Comparative Example 1, the proportion of CAPRIN-1-positive cancer tissue specimens was 52% for breast cancer tissues, 54% for lung cancer, 58% for pancreatic cancer, 55% for renal cancer, 49% for colon cancer, 40% for gastric cancer, 45% for ovarian cancer, and 54% for prostate cancer. Thus, the determination results were largely different from those based on the staining in Example 1.

It was therefore found that the decrease in the staining accuracy due to baking in the paraffin removal process also largely affects the determination result.

Further, when the mouse anti-CAPRIN-1 monoclonal antibody or the rabbit anti-CAPRIN-1 monoclonal antibody prepared in WO 2010/016526, WO 2011/096517, WO 2011/096528, WO 2011/096519, WO 2011/096533, WO 2011/096534, WO 2013/018894, WO 2013/018892, WO 2013/018891, WO 2013/018889, WO 2013/018883, WO 2013/125636, WO 2013/125654, WO 2013/125630, WO 2013/125640, or WO 2015/020212 was applied as the antibody used in the primary antibody reaction step in the present Comparative Example, a decrease in the staining accuracy was also found at the same level as described above.

Example 3: Detection of CAPRIN-1 Protein by Immunohistochemical Staining (2)

Paraffin-embedded sections of various human cancer tissues (manufactured by BIOMAX Inc.) derived from the same specimens as in Example 1 were subjected to immunohistochemical staining in accordance with the description in Example 1, except that the antibody concentration in the primary antibody solution used in the primary antibody reaction step was 0.8 μg/mL, and that the coloring time in the coloring process was about 7 minutes. CAPRIN-1-positive cancer tissue specimens were then determined in the same manner as in Example 2.

Staining images obtained from ovarian cancer tissue are shown in Figure 12. It was found that, even when the different antibody concentration and different coloring time are employed, an ovarian cancer tissue can be stained with an accuracy equivalent to that of the staining images obtained in Example 1 (Figure 11).

According to the determination results, the proportion of CAPRIN-1-positive cancer tissues was 80% for breast cancer tissues, 70% for lung cancer, 80% for pancreatic cancer, 80% for renal cancer, 70% for colon cancer, 55% for gastric cancer, 55% for ovarian cancer, and 80% for prostate cancer. Thus, the determination results were almost the same as those based on the staining in Example 1.

Further, when the mouse anti-CAPRIN-1 monoclonal antibody or the rabbit anti-CAPRIN-1 monoclonal antibody prepared in WO 2010/016526, WO 2011/096517, WO 2011/096528, WO 2011/096519, WO 2011/096533, WO 2011/096534, WO 2013/018894, WO 2013/018892, WO 2013/018891, WO 2013/018889, WO 2013/018883, WO 2013/125636, WO 2013/125654, WO 2013/125630, WO 2013/125640, or WO 2015/020212 was applied as the antibody used in the primary antibody reaction step in the present Example, the proportion of CAPRIN-1-positive cancer tissue specimens among the specimens of each type of cancer tissue was also at the same level as described above.

Example 4: Detection of CAPRIN-1 Protein by Immunohistochemical Staining Method 3

Various paraffin-embedded human cancer tissues (manufactured by BIOMAX Inc.) derived from the same specimens as in Example 1 were subjected to immunohistochemical staining in accordance with the description in Example 1, except that the heat treatment in the antigen retrieval process was carried out at 121°C. CAPRIN-1-positive cancer tissue specimens were then determined in the same manner as in Example 2.

Staining images obtained from ovarian cancer tissue are shown in Figure 13. It was found that, even when a different heating temperature is employed in the antigen retrieval process, staining can be carried out with an accuracy equivalent to that of the staining images obtained in Example 1 (Figure 11).

According to the determination results, the proportion of CAPRIN-1-positive cancer tissue specimens was 77% for breast cancer tissues, 67% for lung cancer, 75% for pancreatic cancer, 75% for renal cancer, 65% for colon cancer, 60% for gastric cancer, 60% for ovarian cancer, and 85% for prostate cancer. Thus, the determination results were almost the same as those based on the staining in Example 1.

Further, when the mouse anti-CAPRIN-1 monoclonal antibody or the rabbit anti-CAPRIN-1 monoclonal antibody prepared in WO 2010/016526, WO 2011/096517, WO 2011/096528, WO 2011/096519, WO 2011/096533, WO 2011/096534, WO 2013/018894, WO 2013/018892, WO 2013/018891, WO 2013/018889, WO 2013/018883, WO 2013/125636, WO 2013/125654, WO 2013/125630, WO 2013/125640, or WO 2015/020212 was applied as the antibody used in the primary antibody reaction step in the present Example, the proportion of CAPRIN-1-positive cancer tissue specimens among the specimens of each type of cancer tissue was also at the same level as described above.

Example 5: Detection of CAPRIN-1 Protein by Immunohistochemical Staining (4)

Paraffin-embedded sections of various human cancer tissues (manufactured by BIOMAX Inc.) derived from the same specimens as in Example 1 were subjected to immunohistochemical staining in accordance with the description in Example 1, except that the heat treatment in the antigen retrieval process was carried out at 121°C, that the antibody concentration in the primary antibody solution used in the primary antibody reaction step was 0.8 $\mu$g/mL, and that the coloring time in the coloring process was about 7 minutes. CAPRIN-1-positive cancer tissue specimens were then determined in the same manner as in Example 2.

Staining images obtained from ovarian cancer tissue are shown in Figure 14. It was found that, even when different heating temperature in the antigen retrieval process, different antibody concentration, and different coloring time are employed, staining can be carried out with an accuracy equivalent to that of the staining images obtained in Example 1 (Figure 11).

According to the determination results, the proportion of CAPRIN-1-positive cancer tissue specimens was 79% for breast cancer tissues, 68% for lung cancer, 77% for pancreatic cancer, 76% for renal cancer, 62% for colon cancer, 62% for gastric cancer, 61% for ovarian cancer, and 86% for prostate cancer. Thus, the determination results were almost the same as those based on the staining in Example 1.

Further, when the mouse anti-CAPRIN-1 monoclonal antibody or the rabbit anti-CAPRIN-1 monoclonal antibody prepared in WO 2010/016526, WO 2011/096517, WO 2011/096528, WO 2011/096519, WO 2011/096533, WO 2011/096534, WO 2013/018894, WO 2013/018892, WO 2013/018891, WO 2013/018889, WO 2013/018883, WO 2013/125636, WO 2013/125654, WO 2013/125630, WO 2013/125640, or WO 2015/020212 was applied as the antibody used in the primary antibody reaction step in the present Example, the proportion of CAPRIN-1-positive cancer tissue specimens among the specimens of each type of cancer tissue was also at the same level as described above.

Example 6: Detection of CAPRIN-1 Protein by Immunohistochemical Staining (5)

Paraffin-embedded sections of various human cancer tissues (manufactured by BIOMAX Inc.) derived from the same specimens as in Example 1 were subjected to immunohistochemical staining in accordance with the description in Example 1, except that the paraffin removal process was carried out as follows. CAPRIN-1-positive cancer tissue specimens were then determined in the same manner as in Example 2.

(Paraffin Removal Process)

From various paraffin-embedded human cancer tissues (manufactured by BIOMAX Inc.), paraffin was removed using Artisan Cleaning Solution (manufactured by Agilent) for 15 minutes. The Artisan Cleaning Solution was replaced twice at 5-minute intervals. This operation was carried out at room temperature. Subsequently, the tissues were washed 3 times with PBS-T for 5 minutes each.

According to the determination results, the proportion of CAPRIN-1-positive cancer tissue specimens was 78% for breast cancer tissues, 66% for lung cancer, 76% for pancreatic cancer, 79% for renal cancer, 63% for colon cancer, 64% for gastric cancer, 62% for ovarian cancer, and 88% for prostate cancer. Thus, the determination results were almost the same as those based on the staining in Example 1.

Further, when the mouse anti-CAPRIN-1 monoclonal antibody or the rabbit anti-CAPRIN-1 monoclonal antibody prepared in WO 2010/016526, WO 2011/096517, WO 2011/096528, WO 2011/096519, WO 2011/096533, WO 2011/096534, WO 2013/018894, WO 2013/018892, WO 2013/018891, WO 2013/018889, WO 2013/018883, WO 2013/125636, WO 2013/125654, WO 2013/125630, WO 2013/125640, or WO 2015/020212 was applied as the antibody used in the primary antibody reaction step in the present Example, the proportion of CAPRIN-1-positive cancer tissue specimens among the specimens of each type of cancer tissue was also at the same level as described above.

Example 7: Detection of CAPRIN-1 Protein by Immunohistochemical Staining (6)

Paraffin-embedded sections of various human cancer tissues (manufactured by BIOMAX Inc.) derived from the same specimens as in Example 1 were subjected to immunohistochemical staining in the same manner as in Example 6, except that the antibody concentration in the primary antibody solution used in the primary antibody reaction step was 0.8 μg/mL, and that the coloring time in the coloring process was about 7 minutes. CAPRIN-1-positive cancer tissue specimens were then determined in the same manner as in Example 2.

According to the determination results, the proportion of CAPRIN-1-positive cancer tissue specimens was 77% for breast cancer tissues, 67% for lung cancer, 72% for pancreatic cancer, 72% for renal cancer, 68% for colon cancer, 58% for gastric cancer, 64% for ovarian cancer, and 82% for prostate cancer. Thus, the determination results were almost the same as those based on the staining in Example 1.

Further, also when the mouse anti-CAPRIN-1 monoclonal antibody or the rabbit anti-CAPRIN-1 monoclonal antibody prepared in WO 2010/016526, WO 2011/096517, WO 2011/096528, WO 2011/096519, WO 2011/096533, WO 2011/096534, WO 2013/018894, WO 2013/018892, WO 2013/018891, WO 2013/018889, WO 2013/018883, WO 2013/125636, WO 2013/125654, WO 2013/125630, WO 2013/125640, or WO 2015/020212 was applied as the antibody used in the primary antibody reaction step in the present Example, the proportion of CAPRIN-1-positive cancer tissue specimens among the specimens of each type of cancer tissue was also at the same level as described above.

Example 8: Detection of CAPRIN-1 Protein by Immunohistochemical Staining Method (7)

Paraffin-embedded sections of various human cancer tissues (manufactured by BIOMAX Inc.) derived from the same specimens as in Example 1 were subjected to immunohistochemical staining in the same manner as in Example 6, except that the heat treatment in the antigen retrieval process was carried out at 121°C. CAPRIN-1-positive cancer tissue specimens were then determined in the same manner as in Example 2.

According to the determination results, the proportion of CAPRIN-1-positive cancer tissue specimens was 80% for breast cancer tissues, 70% for lung cancer, 70% for pancreatic cancer, 78% for renal cancer, 69% for colon cancer, 55% for gastric cancer, 60% for ovarian cancer, and 90% for prostate cancer. Thus, the determination results were almost the same as those based on the staining in Example 1.

Further, when the mouse anti-CAPRIN-1 monoclonal antibody or the rabbit anti-CAPRIN-1 monoclonal antibody prepared in WO 2010/016526, WO 2011/096517, WO 2011/096528, WO 2011/096519, WO 2011/096533, WO 2011/096534, WO 2013/018894, WO 2013/018892, WO 2013/018891, WO 2013/018889, WO 2013/018883, WO 2013/125636, WO 2013/125654, WO 2013/125630, WO 2013/125640, or WO 2015/020212 was applied as the antibody used in the primary antibody reaction step in the present Example, the proportion of CAPRIN-1-positive cancer tissue specimens among the specimens of each type of cancer tissue was also at the same level as described above.

Example 9: Detection of CAPRIN-1 Protein by Immunohistochemical Staining (8)

Paraffin-embedded sections of various human cancer tissues (manufactured by BIOMAX Inc.) derived from the same specimens as in Example 1 were subjected to immunohistochemical staining in the same manner as in Example 6, except that the heat treatment in the antigen retrieval process was carried out at 121°C, that the antibody concentration in the primary antibody solution used in the primary antibody reaction step was 0.8 μg/mL, and that the coloring time in the coloring process was about 7 minutes. CAPRIN-1-positive cancer tissue specimens were then determined in the same manner as in Example 2.

According to the determination results, the proportion of CAPRIN-1-positive cancer tissue specimens was 75% for breast cancer tissues, 65% for lung cancer, 80% for pancreatic cancer, 80% for renal cancer, 62% for colon cancer, 55% for gastric cancer, 65% for ovarian cancer, and 80% for prostate cancer. Thus, the determination results were almost the same as those based on the staining in Example 1.

Further, when the mouse anti-CAPRIN-1 monoclonal antibody or the rabbit anti-CAPRIN-1 monoclonal antibody prepared in WO 2010/016526, WO 2011/096517, WO 2011/096528, WO 2011/096519, WO 2011/096533, WO 2011/096534, WO 2013/018894, WO 2013/018892, WO 2013/018891, WO 2013/018889, WO 2013/018883, WO 2013/125636, WO 2013/125654, WO 2013/125630, WO 2013/125640, or WO 2015/020212 was applied as the antibody used in the primary antibody reaction step in the present Example, the proportion of CAPRIN-1-positive cancer tissue specimens among the specimens of each type of cancer tissue was also at the same level as described above.

From the above results, the staining method of the present invention was found to be capable of detecting the cell membrane protein with high accuracy even when the type of the removal solution and other conditions are changed to some extent.

All publications, patents, and patent applications cited herein are hereby incorporated as they are by the citation.

Description of Symbols

S0101 Embedding medium removal process
S0102 Antigen retrieval process
S0103 Labeling process
S0104 Label detecting process
0210 Embedding medium removal unit
0211 Storage means for removal solution
0212, 0222, 0232, 0234 Contact means
0213 Holding means
0220 Antigen retrieval unit
0221 Antigen retrieval means
0230 Labeling reaction unit
0231 Binding-molecule storage means
0233 Labeled-antibody storage means
0240 Label detecting unit
0241 Detection means
0242 Analysis means
0250 Control unit
0251 CPU
0252 Volatile memory
0253 Nonvolatile memory
0254 Interface
0255 Bus

**Claims**

1.  A method for detecting a cell membrane protein by removing an embedding medium from an embedded fixed tissue, comprising:

    an embedding medium removal process of removing the embedding medium by contacting the fixed tissue comprising an embedding medium in a solid state with a removal solution;
    a labeling process of binding the cell membrane protein of the fixed tissue after the embedding medium removal process to a labeled antibody; and
    a label detecting process of detecting the labeled antibody bound to the cell membrane protein after the labeling process.

2.  The method according to claim 1, wherein the embedding medium removal process is a process of removing the embedding medium by contacting the fixed tissue with the removal solution at a temperature less than the melting point of the embedding medium.

3. The method according to claim 1 or 2, wherein the embedding medium is paraffin.

4. The method according to claim 3, wherein the embedding medium removal process is a process of removing the embedding medium by contacting the fixed tissue with the removal solution while maintaining the fixed tissue at less than 45°C.

5. The method according to any one of claims 1 to 4, wherein the removal solution is a solution comprising a surfactant and/or an organic solvent.

6. The method according to any one of claims 1 to 5, wherein the contact in the embedding medium removal process is immersion.

7. The method according to any one of claims 1 to 6, comprising an antigen retrieval process of retrieving the cell membrane protein in the fixed tissue after the embedding medium removal process.

8. The method according to claim 7, wherein the antigen retrieval process comprises a heating step at 90 to 130°C.

9. The method according to claim 7 or 8, comprising a cooling process of cooling the fixed tissue after the antigen retrieval process.

10. The method according to any one of claims 1 to 9, wherein the labeling process comprises a primary antibody reaction step of reacting a primary antibody having immunological reactivity against the cell membrane protein with the cell membrane protein of the fixed tissue after the embedding medium removal process, and a secondary antibody reaction step of reacting a labeled secondary antibody having immunological reactivity with the primary antibody with the fixed tissue after the primary antibody reaction step.

11. The method according to claim 10, wherein the labeled secondary antibody is a complex of an antibody having immunological reactivity against the primary antibody and peroxidase bound onto a polymer carrier.

12. The method according to claim 11, wherein the label detecting process is a process of detecting the cell membrane protein colored by a coloring reagent reactive with the peroxidase.

13. The method according to claim 11 or 12, wherein the coloring reagent is 3,3'-diaminobenzidine (DAB).

14. The method according to any one of claims 1 to 13, wherein the cell membrane protein is a cell membrane protein expressed on the surface of a cancer cell.

15. The method according to any one of claims 1 to 14, wherein the cell membrane protein is a disease marker.

16. The method according to claim 15, wherein the disease is cancer.

17. The method according to claim 15 or 16, wherein the disease marker is CAPRIN-1 protein.

18. A method for determining a treatment for an individual from whom a fixed tissue is derived, comprising:

a process of detecting disease markers in the embedded fixed tissue using a method according to any one of claims 15 to 17; and
a treatment determining process of determining a treatment for the individual based on the disease markers detected by the process.

19. An apparatus for detecting a cell membrane protein in an embedded fixed tissue, comprising:

an embedding medium removal unit of removing the embedding medium by contacting the fixed tissue comprising an embedding medium in a solid state with a removal solution;
a labeling reaction unit of binding the cell membrane protein of the fixed tissue that has been subjected to the embedding medium removal unit to a labeled antibody; and
a label detecting unit of detecting the labeled antibody bound to the cell membrane protein in the fixed tissue that has been subjected to the labeling reaction unit.

20. The apparatus according to claim 19, wherein the embedding medium removal unit is equipped with a temperature regulating means for making the fixed tissue less than the melting point of the embedding medium.

21. The apparatus according to claim 19 or 20, further comprising an antigen retrieval unit of retrieving the cell membrane protein in the fixed tissue that has been subjected to the embedding medium removal unit.

22. The apparatus according to any one of claims 19 to 21, wherein the cell membrane protein is a cell membrane protein expressed on the surface of a cancer cell.

23. The apparatus according to any one of claims 19 to 22, wherein the cell membrane protein is a disease marker.

24. The apparatus according to claim 23, wherein the disease is cancer.

25. The apparatus according to claim 23 or 24, wherein the disease marker is CAPRIN-1 protein.

# Fig. 1

A

B

# Fig. 2

A

B

# Fig. 3

A

B

# Fig. 4

A

B

# Fig. 5

A

B

# Fig. 6

A

B

# Fig. 7

A

B

# Fig. 8

A

B

# Fig. 9

A

B

# Fig. 10

A

B

# Fig. 11

A

B

# Fig. 12

A

B

# Fig. 13

A

B

# Fig. 14

A

B

# Fig. 15

START

↓

embedding medium
removal process ⎯ S0101

↓

E0101

retrieval of
antigen — YES → antigen retrieval
process ⎯ S0102

NO

↓

labeling process ⎯ S0103

↓

label detecting process ⎯ S0104

↓

END

# Fig. 16

# Fig. 17

## INTERNATIONAL SEARCH REPORT

| International application No. |
|---|
| **PCT/JP2024/012221** |

| A. | CLASSIFICATION OF SUBJECT MATTER |
|---|---|

*G01N 33/48*(2006.01)i
FI:  G01N33/48 P; G01N33/48 R

According to International Patent Classification (IPC) or to both national classification and IPC

| B. | FIELDS SEARCHED |
|---|---|

Minimum documentation searched (classification system followed by classification symbols)

G01N33/48

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2008-527330 A (VENTANA MEDICAL SYST INC.) 24 July 2008 (2008-07-24) claims, paragraph [0003], example 3 | 1-6, 10, 14-16, 18-20, 22-24 |
| Y | | 7-9, 11-13, 17, 21, 25 |
| X | WO 2014/014086 A1 (TORAY INDUSTRIES, INC.) 23 January 2014 (2014-01-23) paragraph [0119] | 1, 3, 5-19, 21-25 |
| Y | | 2, 4, 7-9, 11-13, 17, 20-21, 25 |
| X | JP 2010-501549 A (KOREA RESEARCH INSTITUTE OF BIOSCIENCE AND BIOTECHNOLOGY) 21 January 2010 (2010-01-21) paragraph [0065] | 1-10, 14-16, 18-24 |
| Y | | 2, 4, 7-9, 11-13, 20-21 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 May 2024** | **28 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

| | International application No. |
|---|---|
| | **PCT/JP2024/012221** |

| C. | DOCUMENTS CONSIDERED TO BE RELEVANT | |
|---|---|---|
| Category* | Citation of document, with indication, where appropriate, of the relevant passages | Relevant to claim No. |
| X | WO 2020/138427 A1 (THE UNIVERSITY OF TOKYO) 02 July 2020 (2020-07-02) paragraphs [0081]-[0083] | 1-10, 14-16, 18-24 |
| Y | | 2, 4, 7-9, 11-13, 20-21 |

Form PCT/ISA/210 (second sheet) (July 2022)

## INTERNATIONAL SEARCH REPORT
### Information on patent family members

International application No.

**PCT/JP2024/012221**

| Patent document cited in search report | | | Publication date (day/month/year) | Patent family member(s) | | | Publication date (day/month/year) |
|---|---|---|---|---|---|---|---|
| JP | 2008-527330 | A | 24 July 2008 | US | 2006/0252025 | A1 | |
| | | | | claims, paragraph [0005], example 3 | | | |
| WO | 2014/014086 | A1 | 23 January 2014 | US | 2015/0185222 | A1 | |
| | | | | paragraph [0118] | | | |
| | | | | EP | 2876447 | A1 | |
| | | | | CN | 104471404 | A | |
| | | | | KR | 10-2015-0037752 | A | |
| JP | 2010-501549 | A | 21 January 2010 | US | 2010/0196350 | A1 | |
| | | | | paragraph [0086] | | | |
| | | | | WO | 2008/023947 | A1 | |
| | | | | KR | 10-2008-0018149 | A | |
| | | | | CN | 101605560 | A | |
| WO | 2020/138427 | A1 | 02 July 2020 | US | 2022/0073641 | A1 | |
| | | | | paragraph [0111] | | | |
| | | | | EP | 3903790 | A1 | |
| | | | | CN | 113226329 | A | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010016527 A
- WO 2010016526 A
- WO 2011025442 A
- WO 2004077057 A
- JP 2023050776 A
- WO 2011096517 A
- WO 2011096528 A
- WO 2011096519 A
- WO 2011096533 A
- WO 2011096534 A
- WO 2011096535 A
- WO 2013018886 A
- WO 2013018894 A
- WO 2013018892 A
- WO 2013018891 A
- WO 2013018889 A
- WO 2013018883 A
- WO 2013125636 A
- WO 2013125654 A
- WO 2013125630 A
- WO 2013125640 A
- WO 2013147169 A
- WO 2013147176 A
- WO 2015020212 A
- WO 2018079740 A
- WO 2019189780 A

**Non-patent literature cited in the description**

- *Lancet Oncol*, 2016, vol. 17, e256-62
- *Pharm Res*, November 2015, vol. 32 (11), 3526-40
- **HOPWOOD D**. Fixatives and fixation: a review. *Histochem J.*, 1969, vol. 1 (4), 323-60
- Immunohistochemical Staining Methods. DAKO North America, Inc, 2013
- **SHI et al.** *J Histochemistry & Cytochemistry*, 2011, vol. 59, 13-32
- **D'AMICO et al.** *J Immunological Methods*, 2009, vol. 341, 1-18
- **MCNICOLL** ; **RICHMOND**. *Histopathology*, 1998, vol. 32, 97-103